(19) Europäisches Patentamt
European Patent Office
Office européen des brevets

(11) EP 1 028 935 B1

(12) EUROPÄISCHE PATENTSCHRIFT

(45) Veröffentlichungstag und Bekanntmachung des Hinweises auf die Patenterteilung:
**16.03.2005 Patentblatt 2005/11**

(21) Anmeldenummer: **98961141.3**

(22) Anmeldetag: **05.11.1998**

(51) Int Cl.[7]: **C07C 43/225**, C09K 19/32, C07C 25/13, C07F 5/02, C07C 37/01, C07C 25/24

(86) Internationale Anmeldenummer:
**PCT/EP1998/007080**

(87) Internationale Veröffentlichungsnummer:
**WO 1999/024385 (20.05.1999 Gazette 1999/20)**

(54) **FLUORIERTE DERIVATE DES PHENANTHRENS UND IHRE VERWENDUNG IN FLÜSSIGKRISTALLMISCHUNGEN**

FLUORINATED DERIVATIVES OF PHENANTHRENE AND THE UTILIZATION THEREOF IN LIQUID CRYSTAL MIXTURES

DERIVES FLUORES DE PHENANTHRENE ET LEUR UTILISATION DANS DES MELANGES A CRISTAUX LIQUIDES

(84) Benannte Vertragsstaaten:
**DE FR GB**

(30) Priorität: **05.11.1997 DE 19748819**

(43) Veröffentlichungstag der Anmeldung:
**23.08.2000 Patentblatt 2000/34**

(73) Patentinhaber: **Aventis Research & Technologies GmbH & Co KG**
**65926 Frankfurt am Main (DE)**

(72) Erfinder: **WINGEN, Rainer**
**D-65795 Hattersheim (DE)**

(74) Vertreter: **Isenbruck, Günter, Dr. et al**
**Isenbruck, Bösl, Hörschler, Wichmann, Huhn**
**Patentanwälte**
**Theodor-Heuss-Anlage 12**
**68165 Mannheim (DE)**

(56) Entgegenhaltungen:
**DE-A- 4 432 970** **DE-A- 19 500 768**

- **PATENT ABSTRACTS OF JAPAN vol. 98, no. 14, 31. Dezember 1998 & JP 10 236992 A (CHISSO CORP.), 8. September 1998**
- **TURNER W.R.: "3-ethenyl, 3-ethynyl, 3-aryl, and 3-cyclopropyl-2,4,5-trifluo- robenzoic acids: useful intermediates in the synthesis of quinolone antibacterials" TETRAHEDRON LETTERS., Bd. 34, Nr. 2, 1993, Seiten 281-284, XP002096375 OXFORD GB**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Neben nematischen und cholesterischen Flüssigkristallen werden in jüngerer Zeit auch optisch aktive geneigt smektische (ferroelektrische) Flüssigkristalle in kommerziellen Displayvorrichtungen verwendet.

Clark und Lagerwall konnten zeigen, daß der Einsatz ferroelektrischer Flüssigkristalle (FLC) in sehr dünnen Zellen zu optoelektrischen Schalt- oder Anzeigeelementen führt, die im Vergleich zu den herkömmlichen TN ("twisted nematic")-Zellen um bis zu einem Faktor 1000 schnellere Schaltzeiten haben (siehe z.B. EP-A 0 032 362). Aufgrund dieser und anderer günstiger Eigenschaften, z.B. der bistabilen Schaltmöglichkeit und des nahezu blickwinkelunabhängigen Kontrasts, sind FLCs grundsätzlich für Anwendungsgebiete wie Computerdisplays gut geeignet.

Für die Verwendung von FLCs in elektrooptischen oder vollständig optischen Bauelementen benötigt man entweder Verbindungen, die geneigte bzw. orthogonale smektische Phasen ausbilden und selbst optisch aktiv sind, oder man kann durch Dotierung von Verbindungen, die zwar solche smektischen Phasen ausbilden, selbst aber nicht optisch aktiv sind, mit optisch aktiven Verbindungen ferroelektrische smektische Phasen induzieren. Die gewünschte Phase soll dabei über einen möglichst großen Temperaturbereich stabil sein.

Zur Erzielung eines guten Kontrastverhältnisses in elektrooptischen Bauelementen ist eine einheitliche planare Orientierung der Flüssigkristalle nötig. Eine gute Orientierung in der $S_A$ und $S^*_C$-Phase läßt sich z.B. erreichen, wenn die Phasenfolge der Flüssigkristallmischung mit abnehmender Temperatur lautet:

$$\text{Isotrop} \rightarrow N^* \rightarrow S_A \rightarrow S_C^*$$

Voraussetzung ist, daß der Pitch (Ganghöhe der Helix) in der $N^* \rightarrow$ Phase sehr groß (größer 10µm) oder, noch besser, völlig kompensiert ist (siehe z.B. T. Matsumoto et al., Proc. of the 6th Int. Display Research Conf., Japan Display, Sept. 30 - Okt. 2, 1986, Tokyo, Japan, S. 468-470; M. Murakami et al., ibid. S. 344 -S. 347). Dies erreicht man z.B., indem man zu der chiralen Flüssigkristallmischung, die in der $N^*$-Phase z.B. eine linksdrehende Helix aufweist, einen oder mehrere optisch aktive Dotierstoffe, die eine rechtsdrehende Helix induzieren, in solchen Mengen hinzugibt, daß die Helix kompensiert wird.

Für die Verwendung des SSFLCD-Effektes (Surface Stabilized Ferroelectric Liquid Crystal Display) von Clark und Lagerwall zur einheitlichen, planaren Orientierung ist ferner Voraussetzung, daß der Pitch in der smektischen C*-Phase wesentlich größer ist als die Dicke des Anzeigeelementes (Mol. Cryst. Liq. Cryst. 1983, 94, 213 und 1984, 114, 151.

Die optische Schaltzeit $\tau$ [µs] ferroelektrischer Flüssigkristallsysteme, die möglichst kurz sein soll, hängt von der Rotationsviskosität des Systems $\gamma$[mPas], der spontanen Polarisation $P_s$[nC/cm$^2$] und der elektrischen Feldstärke E[V/m] ab nach der Beziehung

$$\tau \sim \frac{\gamma}{P_s \cdot E}$$

Da die Feldstärke E durch den Elektrodenabstand im elektrooptischen Bauteil und durch die angelegte Spannung festgelegt ist, muß das ferroelektrische Anzeigemedium niedrigviskos sein und eine hohe spontane Polarisation aufweisen, damit eine kurze Schaltzeit erreicht wird.

Schließlich wird neben thermischer, chemischer und photochemischer Stabilität eine kleine optische Anisotropie $\Delta n$ und eine geringe positive oder vorzugsweise negative dielektrische Anisotropie $\Delta\varepsilon$ verlangt (siehe z.B. S.T. Lagerwall et al., "Ferroelectric Liquid Crystals for Displays" SID Symposium, Oct. Meeting 1985, San Diego, Ca, USA).

Die Gesamtheit dieser Forderungen ist nur mit Mischungen aus mehreren Komponenten zu erfüllen. Als Basis (oder Matrix) dienen dabei bevorzugt Verbindungen, die möglichst selbst bereits die gewünschte Phasenfolge $I \rightarrow N \rightarrow S_A \rightarrow S_C$ aufweisen. Weitere Komponenten der Mischung werden oftmals zur Schmelzpunktserniedrigung und zur Verbreiterung der $S_C$-und meist auch N-Phase, zum Induzieren der optischen Aktivität, zur Pitch-Kompensation und zur Anpassung der optischen und dielektrischen Anisotropie zugesetzt, wobei aber beispielsweise die Rotationsviskosität möglichst nicht vergrößert werden soll.

Ferroelektrische Flüssigkristallanzeigen lassen sich auch durch Nutzung des DHF (Distorted Helix Formation) -Effektes oder des PSFLCD-Effektes (Pitch Stabilized Ferroelectric Liquid Crystal Display, auch SBF = Short Pitch Bistable Ferroelectric Effect genannt) betreiben. Der DHF-Effekt wurde von B.I. Ostrovski in Advances in Liquid Crystal Research and Applications, Oxford/Budapest 1980, 469ff. beschrieben, der PSFLCD-Effekt ist in DE-A 39 20 625 bzw. EP-A 0 405 346 beschrieben. Zur Nutzung dieser Effekte wird im Gegensatz zum SSFLCD-Effekt ein flüssigkristallines Material mit einem kurzen $S_c$-Pitch benötigt.

Fluorierte Derivate des Phenanthrens zur Verwendung in Flüssigkristallmischungen sind beispielsweise aus

der DE-A 195 00 768 bekannt.

Da aber die Entwicklung, insbesondere von ferroelektrischen Flüssigkristallmischungen, noch in keiner Weise als abgeschlossen betrachtet werden kann, sind die Hersteller von Displays an den unterschiedlichsten Komponenten für Mischungen interessiert. Dieses u.a. auch deshalb, weil erst das Zusammenwirken der flüssigkristallinen Mischungen mit den einzelnen Bauteilen der Anzeigevorrichtung bzw. der Zellen (z.B. der Orientierungsschicht) Rückschlüsse auf die Qualität auch der flüssigkristallinen Mischungen zuläßt.

Aufgabe der vorliegenden Erfindung war es daher, neue Verbindungen bereitzustellen, die in flüssigkristallinen Mischungen geeignet sind, das Eigenschaftsprofil dieser Mischungen zu verbessern.

Es wurde nun überraschend gefunden, das fluorierte Phenanthren-Derivate der Formel (I) in besonderer Weise zum Einsatz in Flüssigkristallmischungen geeignet sind.

Gegenstand der Erfindung sind daher fluorierte Phenanthren-Derivate der Formel (I),

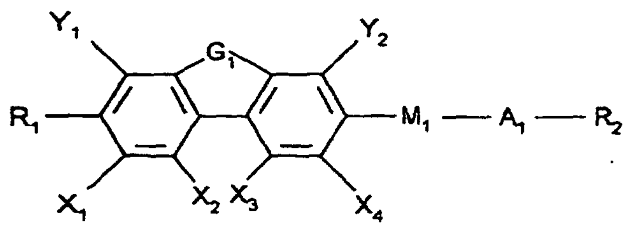

**(I)**

wobei die Symbole und Indizes folgende Bedeutungen haben:

G1 : -CH=CH- oder $-CH_2CH_2-$

$X_1,X_2,X_3,X_4$ : unabhängig voneinander H oder F , mit den Maßgaben, daß

a) $X_1$ und $X_2$ und $X_3$ urid $X_4$ nicht zugleich H bedeuten
b) $X_1$ und $X_2$ und $X_3$ und $X_4$ nicht zugleich F bedeuten
c) mindestens ein X aus dieser Gruppe F bedeutet;

$Y_2$: H oder F; $Y_1$ : F;

$R^1$, $R^2$ sind gleich oder verschieden

a) ein geradkettiger oder verzweigter Alkylrest (mit oder ohne asymmetrisches C-Atom) mit 1 bis 20 C-Atomen, worin auch ein oder mehrere H-Atome durch F substituiert sein können und wobei

a1) eine oder mehrere nicht benachbarte und nicht terminale $CH_2$-Gruppen durch -O-, -S-, -CO-O-, -O-CO-, -O-CO-O- oder $-Si(CH_3)_2-$ ersetzt sein können und/oder
a2) eine oder mehrere $CH_2$-Gruppen durch -CH=CH-; -C≡C-, Cyclopropan-1,2-diyl, 1,4-Phenylen, 1,4-Cyclohexylen oder 1,3-Cyclopentylen ersetzt sein können und/oder
a3) die terminale $CH_3$-Gruppe durch eine der folgenden chiralen Gruppen (optisch aktiv oder racemisch) ersetzt sein kann:

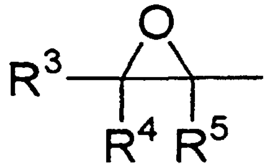

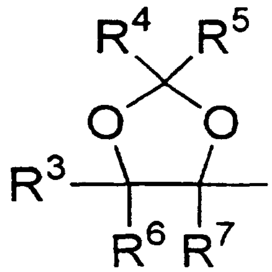

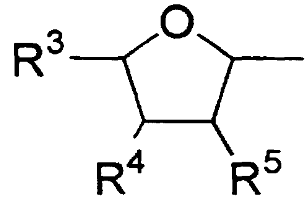

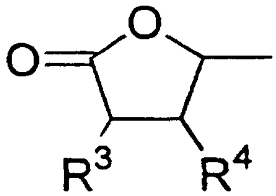

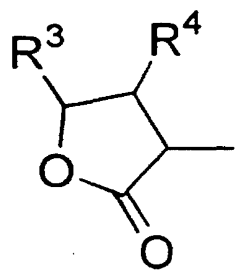

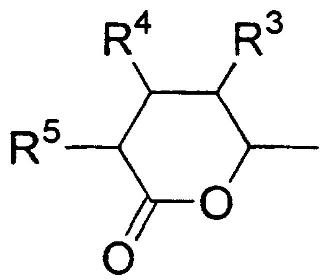

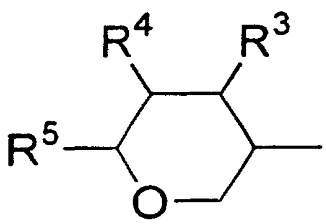

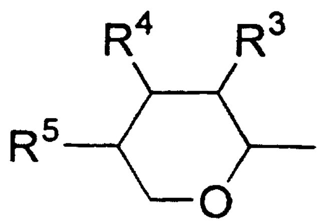

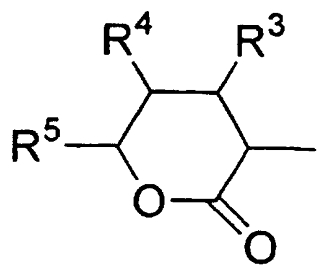

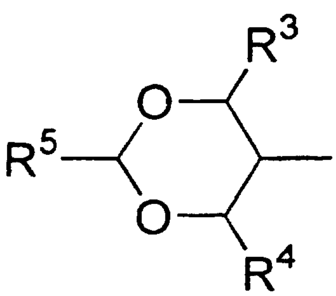

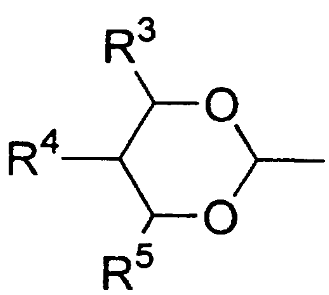

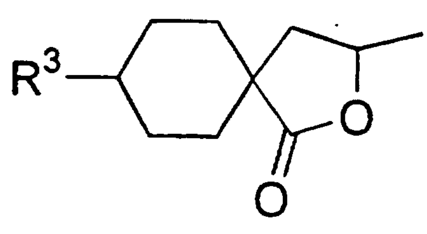

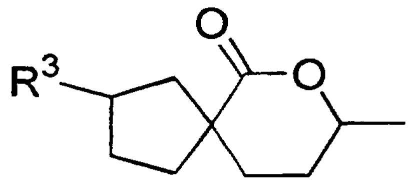
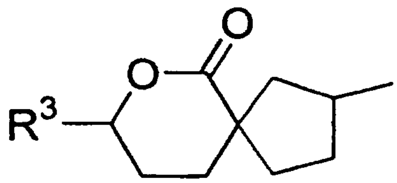
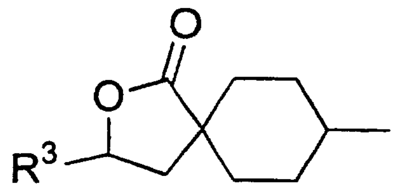

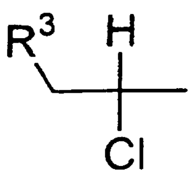
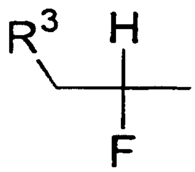
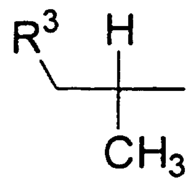
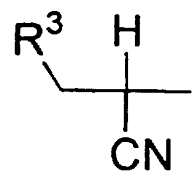

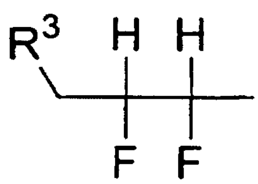

$R^1$ auch Wasserstoff, $-OCF_3$, $-CF_3$, -CN, -F, -Cl, $-OCHF_2$, $-OCH_2F$, $-CHF_2$ oder $-CH_2F$;
b) $R^1$ auch Wasserstoff, Cl oder F

$R^3$, $R^4$, $R^5$, $R^6$, $R^7$ sind gleich oder verschieden

a) Wasserstoff
b) ein geradkettiger oder verzweigter Alkylrest (mit oder ohne asymmetrisches Kohlenstoffatom) mit 1 bis 16 C-Atomen, wobei

b1) eine oder mehrere nicht benachbarte und nicht terminale $CH_2$-Gruppen durch -O- ersetzt sein können und/oder
b2) eine oder zwei $CH_2$-Gruppen durch -CH=CH- ersetzt sein können,

c) $R^4$ und $R^5$ zusammen auch $-(CH_2)_4-$ oder $-(CH_2)_5-$ , wenn sie an ein Oxiran-, Dioxolan-, Tetrahydrofuran-, Tetrahydropyran-, Butyrolacton- oder Valerolacton-System gebunden sind;

mit der Maßgabe, daß $R^3$ nur dann Wasserstoff sein kann, wenn $R^3$ Substituent eines der aufgeführten Ringsysteme ist;

$M^1$ -CO-O-, $-CH_2$-O-, -CH=CH-, -C≡C-, $-CH_2-CH_2$-CO-O-, $-CH_2CH_2CH_2CH_2-$ , $CH_2CH_2CH_2O$- und deren spiegelbildliche Anordnungen; oder eine Einfachbindung;

$A^1$ 1,4-Phenylen, wobei ein oder mehrere H-Atome durch F, Cl und/oder CN ersetzt sein können, 1,4-Cyclohexylen, wobei ein oder zwei H-Atome durch CN und/oder $CH_3$ und/oder F ersetzt sein

können, Naphthalin-2,6-diyl, wobei ein oder mehrere H-Atome durch F, Cl und/oder CN ersetzt sein können ; oder eine Einfachbindung.

Bevorzugt haben die Symbole und Indizes folgende Bedeutungen haben:

G1 : $-CH=CH-$ oder $-CH_2CH_2-$
$X_2,X_3,X_4$ : unabhängig voneinander H oder F,
$Y_2$ : H oder F;
$R^1$, $R^2$ sind gleich oder verschieden

a) ein geradkettiger oder verzweigter Alkylrest (mit oder ohne asymmetrisches C-Atom) mit 1 bis 20 C-Atomen, worin auch ein oder mehrere H-Atome durch F substituiert sein kann und wobei

a1) eine oder mehrere nicht benachbarte und nicht terminale $CH_2$-Gruppen durch -O-, -S-, -CO-O-, -O-CO-, -O-CO-O- oder $-Si(CH_3)_2-$ ersetzt sein können und/oder
a2) eine oder mehrere $CH_2$-Gruppen durch -CH=CH-, -C≡C-, Cyclopropan-1,2-diyl, 1,4-Phenylen, 1,4-Cyclohexylen oder 1,3-Cyclopentylen ersetzt sein können und/oder
a3) die terminale $CH_3$-Gruppe durch eine der folgenden chiralen Gruppen (optisch aktiv oder racemisch) ersetzt sein kann:

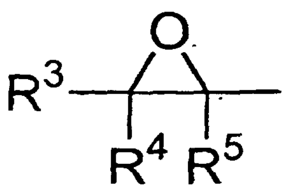
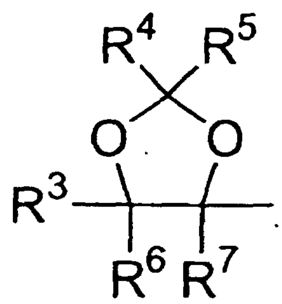
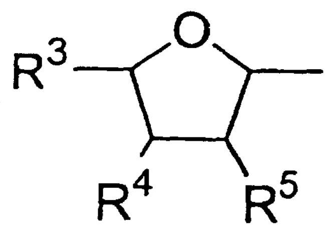
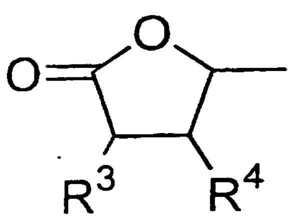
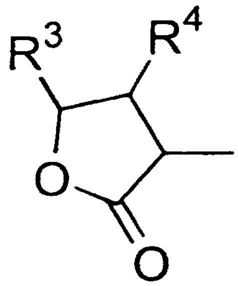
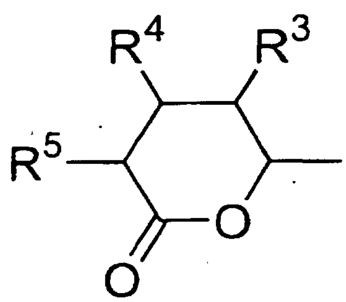

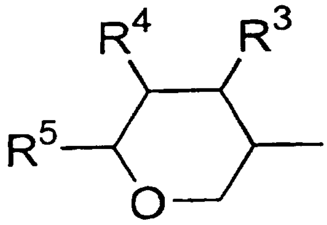

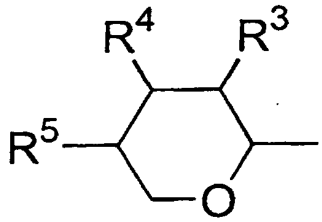

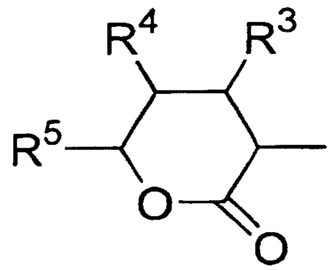

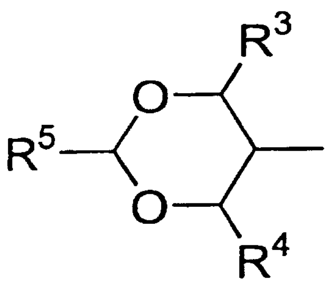

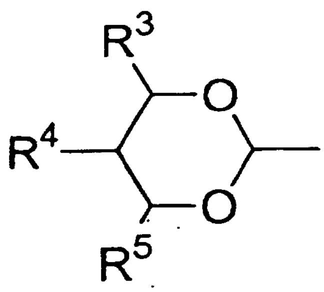

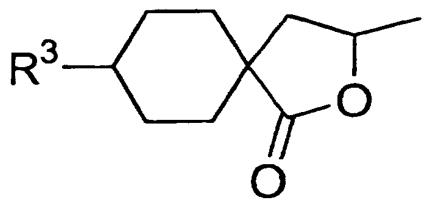

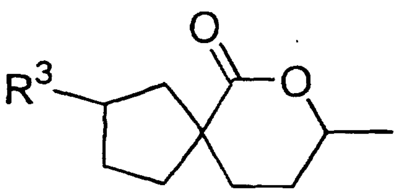

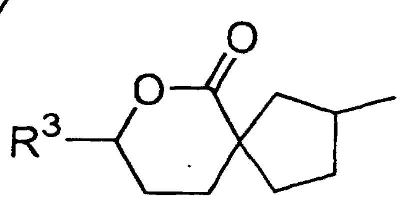

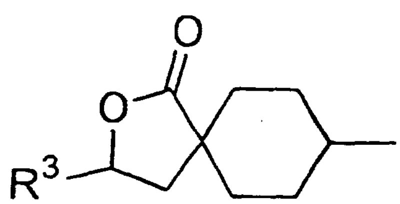

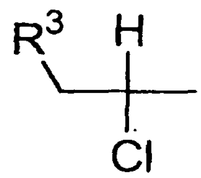 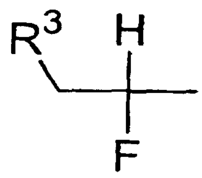 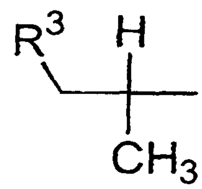 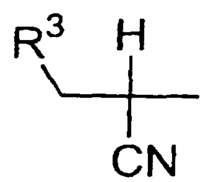

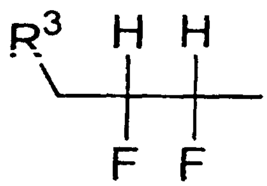

$R^1$ auch Wasserstoff, $-OCF_3$, $-CF_3$, -CN, -F, -Cl, $-OCHF_2$, $-OCH_2F$, $-CHF_2$ oder $-CH_2F$;

b) $R^1$ auch Wasserstoff, Cl oder F

$R^3$, $R^4$, $R^5$, $R^6$, $R^7$ sind gleich oder verschieden

a) Wasserstoff

b) ein geradkettiger oder verzweigter Alkylrest (mit oder ohne asymmetrisches Kohlenstoffatom) mit 1 bis 16 C-Atomen, wobei

b1) eine oder mehrere nicht benachbarte und nicht terminale $CH_2$-Gruppen durch -O- ersetzt sein können und/oder

b2) eine oder zwei $CH_2$-Gruppen durch -CH=CH- ersetzt sein können,

c) $R^4$ und $R^5$ zusammen auch $-(CH_2)_4-$ oder $-(CH_2)_5-$, wenn sie an ein Oxiran-, Dioxolan-, Tetrahydrofuran-, Tetrahydropyran-, Butyrolactonoder Valerolacton-System gebunden sind;

mit der Maßgabe, daß $R^3$ nur dann Wasserstoff sein kann, wenn $R^3$ Substituent eines der aufgeführten Ringsysteme ist;

$M^1$ eine Einfachbindung;
$A^1$ eine Einfachbindung.
$X_1$, $Y_1$, Fluor

mit den Maßgaben, dass

i) $Y_2$, $X_2$, $X_3$, $X_4$ Wasserstoff sind, oder

ii) $Y_2$, $X_2$ Fluor und $X_3$, $X_4$ Wasserstoff sind, oder

iii) $Y_2$, $R_1$ Fluor und $X_2$, $X_3$, $X_4$ Wasserstoff sind, oder

iiii) $X_2$ Fluor ist und $Y_2$, $X_3$, $X_4$ Wasserstoff sind, $G_1$-CH = CH-ist, $R_1$H, Cl, F, $CF_3O$, $CH_2HO$, $CF_3$, ein geradkettiger

Alkylrest mit 1 bis 5 C-Atomen und $R_2$ ein geradkettiger Alkyl- oder Alkyloxyrest mit 1 bis 5 C-Atomen ist.

Mit der Bereitstellung von Verbindungen der Formel (I) wird ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

In diesem Zusammenhang besitzen die Verbindungen der Formel (I) einen breiten Anwendungsbereich. In Abhängigkeit von der Auswahl der Substituenten können sie als Basismaterialien dienen, aus denen flüssigkristalline Phasen zum überwiegenden Teil zusammengesetzt sind; es können aber auch Verbindungen.der Formel (I) flüssigkristallinen Basismaterialien aus anderen Verbindungsklassen zugesetzt werden, um beispielsweise die dielektrische und/oder optische Anisotropie eines solchen. Dielektrikums zu beeinflussen und/oder um dessen Schwellenspannung und/oder dessen Viskosität zu optimieren.

Besonders geeignet sind die Verbindungen der Formel (I), um schon in geringen Zumischmengen die dielektrische Anisotropie ($\Delta\varepsilon$) zu beeinflussen.

Unter Ausnutzung dieser Eigenschaft eignen sich die erfindungsgemäßen Verbindungen der Formel (I) insbesondere zur Verwendung in FLC-Mischungen, die im *Inverse Mode* betrieben werden. Sie eignen sich weiterhin besonders zur Verwendung in Mischungen, die auf der ECB-Mode *(electrically controlled birefringence)* basieren. Auch für die Verwendung in nematischen und chiralsmektischen Mischungen, die für *active matrix bzw. super-TFT*- Modi entwickelt werden, sind die Verbindungen der Formel (I) in besonderem Maße geeignet.

Die Herstellung der erfindungsgemäßen Verbindungen erfolgt nach an sich literaturbekannten Methoden, wie sie in Standardwerken zur Organischen Synthese, z. B. Houben-Weyl, Methoden der Organischen Chemie, Georg-Thieme-Verlag, Stuttgart, beschrieben werden.

Die Herstellung erfolgt dabei unter Reaktionsbedingungen, die für die genannten Umsetzungen bekannt und geeignet sind. Dabei kann auch von an sich bekannten, hier nicht näher erwähnten Varianten Gebrauch machen.

Die Ausgangsstoffe können gewünschtenfalls auch in situ gebildet werden, und zwar derart, daß man sie aus dem Reaktionsgemisch nicht isoliert, sondern sofort weiter zu den Verbindungen der Formel (I) umsetzt.

Beispielhaft sind in den folgenden Schemata ein mögliche Synthesewege für die Verbindungen der Formel (I) angegeben, wobei auch andere Verfahren denkbar und möglich sind.

**Schema 1**

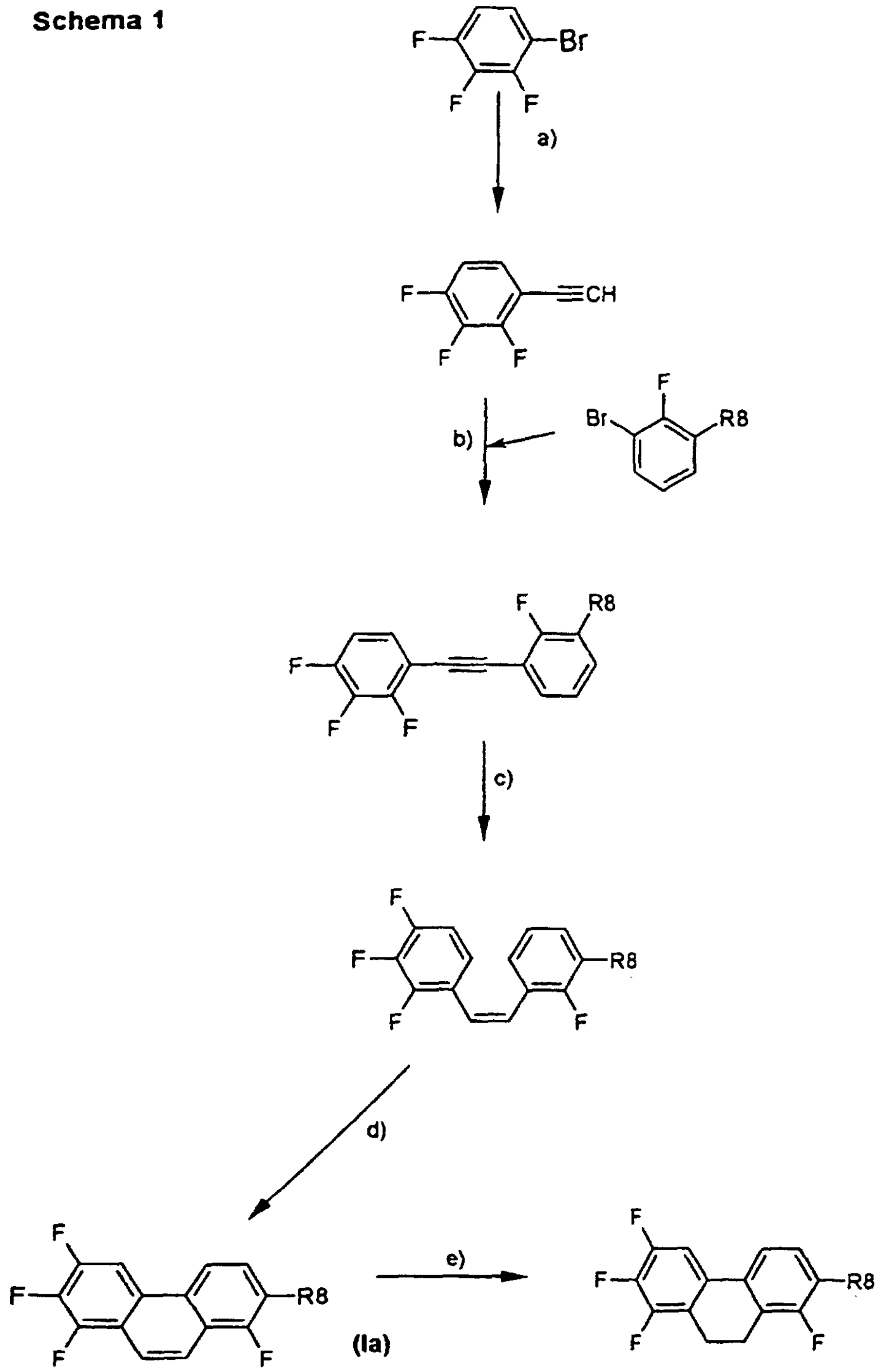


(Ia)

(Ib)

a) H-C≡C-TMS, HN(iPr)2, Pd(II) / Cu(I)-Katalysator ; z.B. J.Organomet,Chem.453 (1993) 2, C19

b) $HN(iPr)_2$, Pd(0)-Katalysator; analog J.Organomet.Chem. 453 (1993) 2, C19

c) $H_2$ /Katalysator (P.N.Rylander, Hydrogenation Methods, Academic Press, London, 1985, p.53)

d) Photocyclisierung (z.B. J.Am.Chem.Soc. 84, 4361 (1962))

e) $H_2$ / Pd (C )

$R^8$: entspricht $R^2$ bzw. kann durch geeignete nachgeschaltete Reaktionen in $R^2$ überführt werden

Das lt.Schema 1 für die Synthese von (Ia) bzw. (Ib) eingesetzte 2,3,4-Trifluorbrombenzol ist käuflich erhältlich; die Synthese des 3-Brom-2 fluor-R8-phenylderivates ist nachstehend beschrieben. Auf der Stufe der Photocyclisierung des 1 -(2,3,4-Trifluorphenyl)-2-(2-Fluor-3-R8-phenyl)ethens wird vorzugsweise das (Z)-Isomer eingesetzt; es kann jedoch auch das (E)-Isomer eingesetzt werden, da daraus unter den Bedingungen der Photocyclisierung das (Z)-Isomer entstehen und in situ zu (Ia) reagieren kann.

Gem. Schema 2 können aus 1-Brom-2,4,5-trifluorbenzol (käuflich erhältlich) und oben erwähntem 3-Brom-2-fluor-R8-phenylderivat die Zielstrukturen (Ic), (Id), (Ie) und (If) erhalten werden.
In Analogie beschreibt Schema 3, ausgehend von dem ebenfalls käuflich erhältlichen 1 -Brom-2,4-difluorbenzol, die Synthese der Zielverbindungen (Ig), (Ih), (Ii) und (Ik).
Das zu Beginn des Schema 4 aufgeführte 1-Brom-2,3,4-trifluorbenzol ist käuflich erhältlich; die Reaktionen verlaufen im wesentlichen analog zu den bereits beschriebenen in den Schemata 1-3.

Schema 2

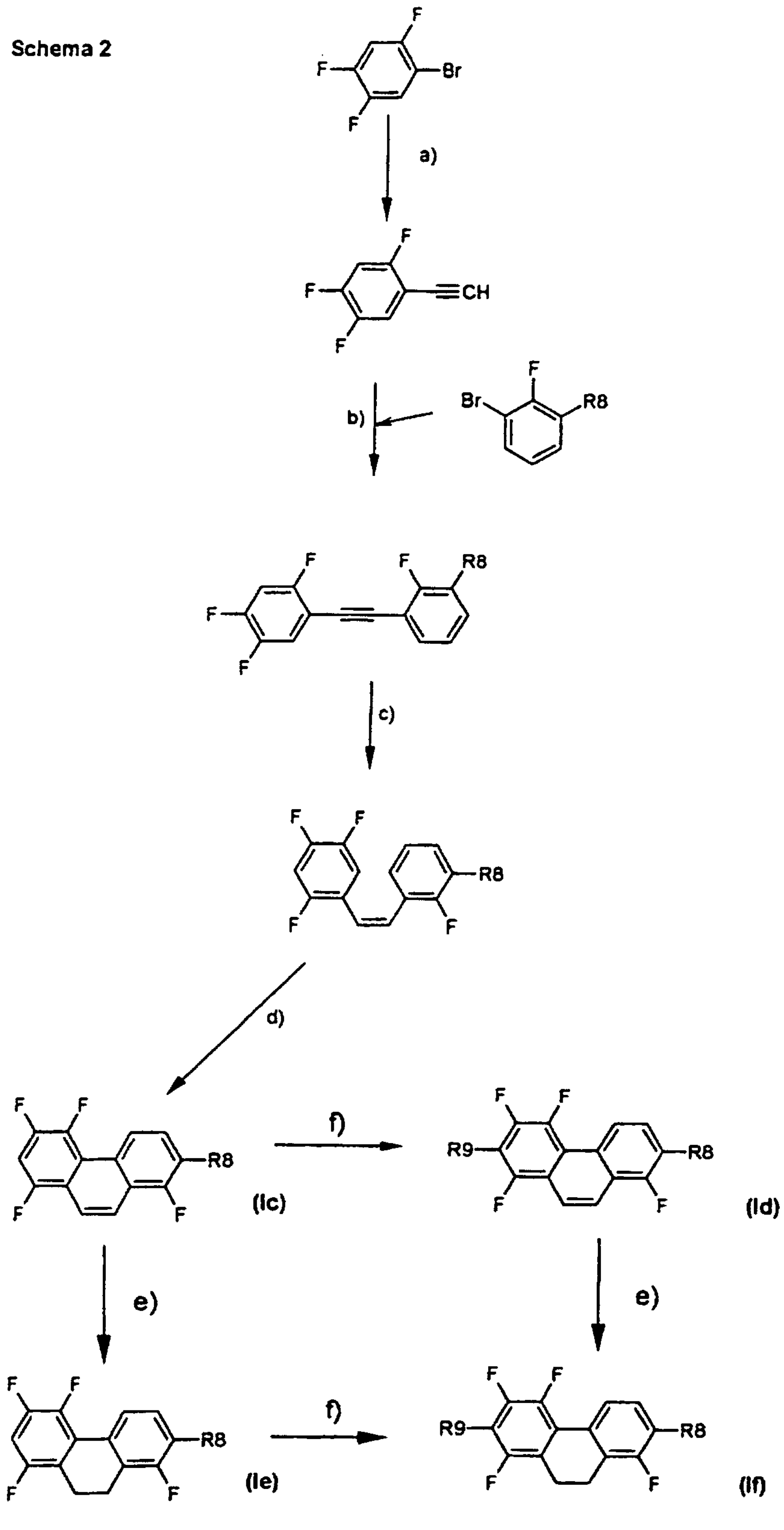


(Ic) (Id) (Ie) (If)

a) H-C≡C-TMS, $HN(iPr)_2$, Pd(II) / Cu(I)-Katalysator ; z.B. J.Organomet,Chem.453 (1993) 2, C19

b) $HN(iPr)_2$, Pd(0)-Katalysator; analog J.Organomet.Chem. 453 (1993) 2, C19

c) $H_2$ /Katalysator (P.N.Rylander, Hydrogenation Methods, Academic Press, London, 1985, p.53)

d) Photocyclisierung (z.B. J.Am.Chem.Soc. 84, 4361 (1962))

e) $H_2$ / Pd (C )

f) 1. LDA 2. $R^9$-Br oder 1.LDA 2.R-CHO 3. H+ 4. $H_2$

$R^8$: entspricht $R^2$ bzw. kann durch geeignete nachgeschaltete Reaktionen in $R^2$ überführt werden

$R^9$ : entspricht $R^1$ bzw. kann durch geeeignete nachgeschaltete Reaktionen in $R^1$ überführt werden

R : entspricht dem um 1 C-Atom kürzeren $R^9$

Schema 3

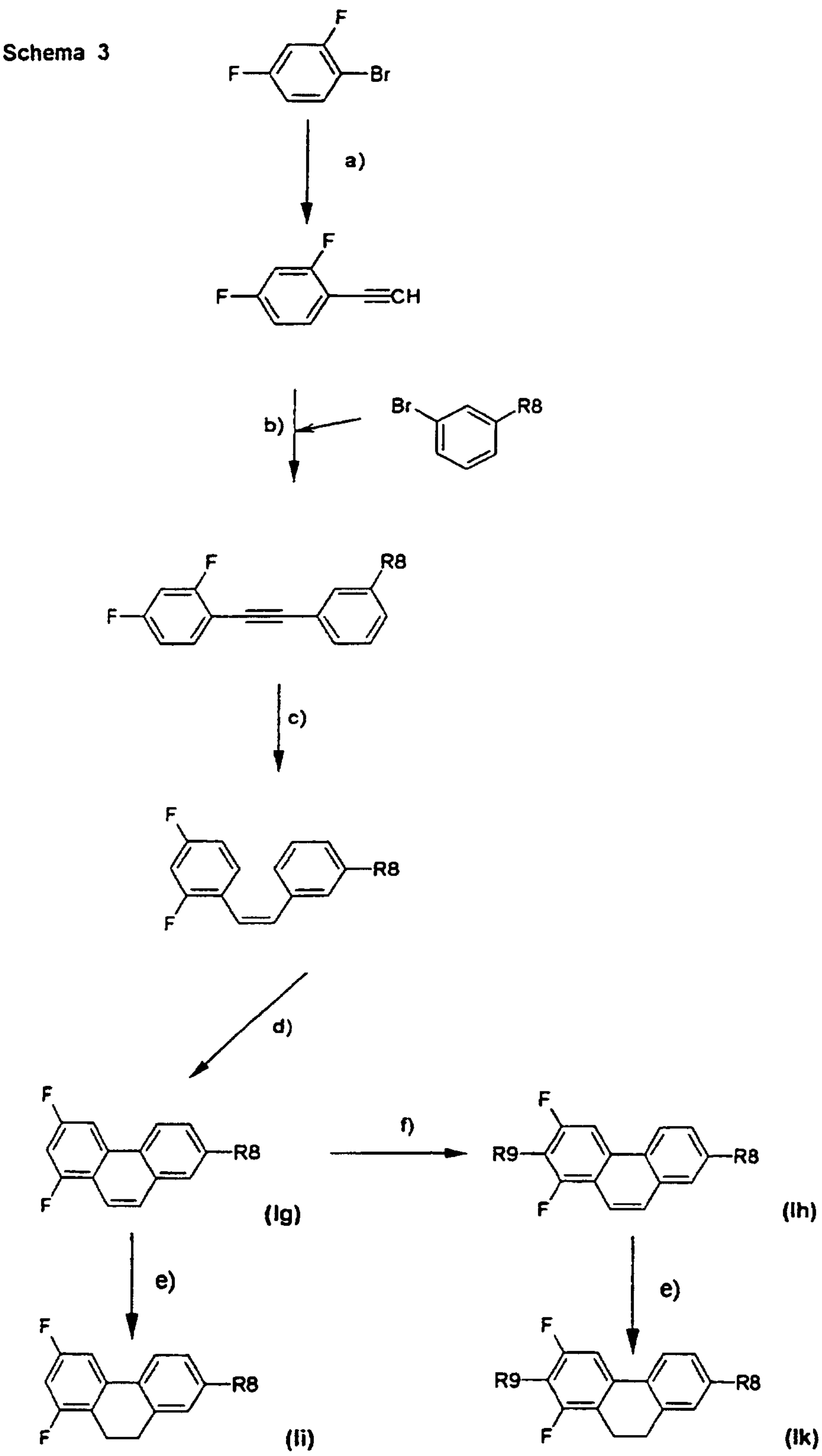


(Ig)

(Ih)

(Ii)

(Ik)

a) H-C≡C-TMS, $HN(iPr)_2$, Pd(II) / Cu(I)-Katalysator ; z.B. J.Organomet,Chem.453 (1993) 2, C19

b) $HN(iPr)_2$, Pd(0)-Katalysator; analog J.Organomet.Chem. 453 (1993) 2, C19

c) $H_2$ /Katalysator (P.N.Rylander, Hydrogenation Methods, Academic Press, London, 1985, p.53)

d) Photocyclisierung (z.B. J.Am.Chem.Soc. 84, 4361 (1962))

e) $H_2$ / Pd (C )

f) 1. LDA 2. $R^9$-Br oder 1.LDA 2.R-CHO 3. H+ 4. $H_2$

$R^8$: entspricht $R^2$ bzw. kann durch geeignete nachgeschaltete Reaktionen in $R^2$ überführt werden

$R^9$ : entspricht $R^1$ bzw. kann durch geeeignete nachgeschaltete Reaktionen in $R^1$ überführt werden

R : entspricht dem um 1 C-Atom kürzeren $R^9$

**Schema 4**

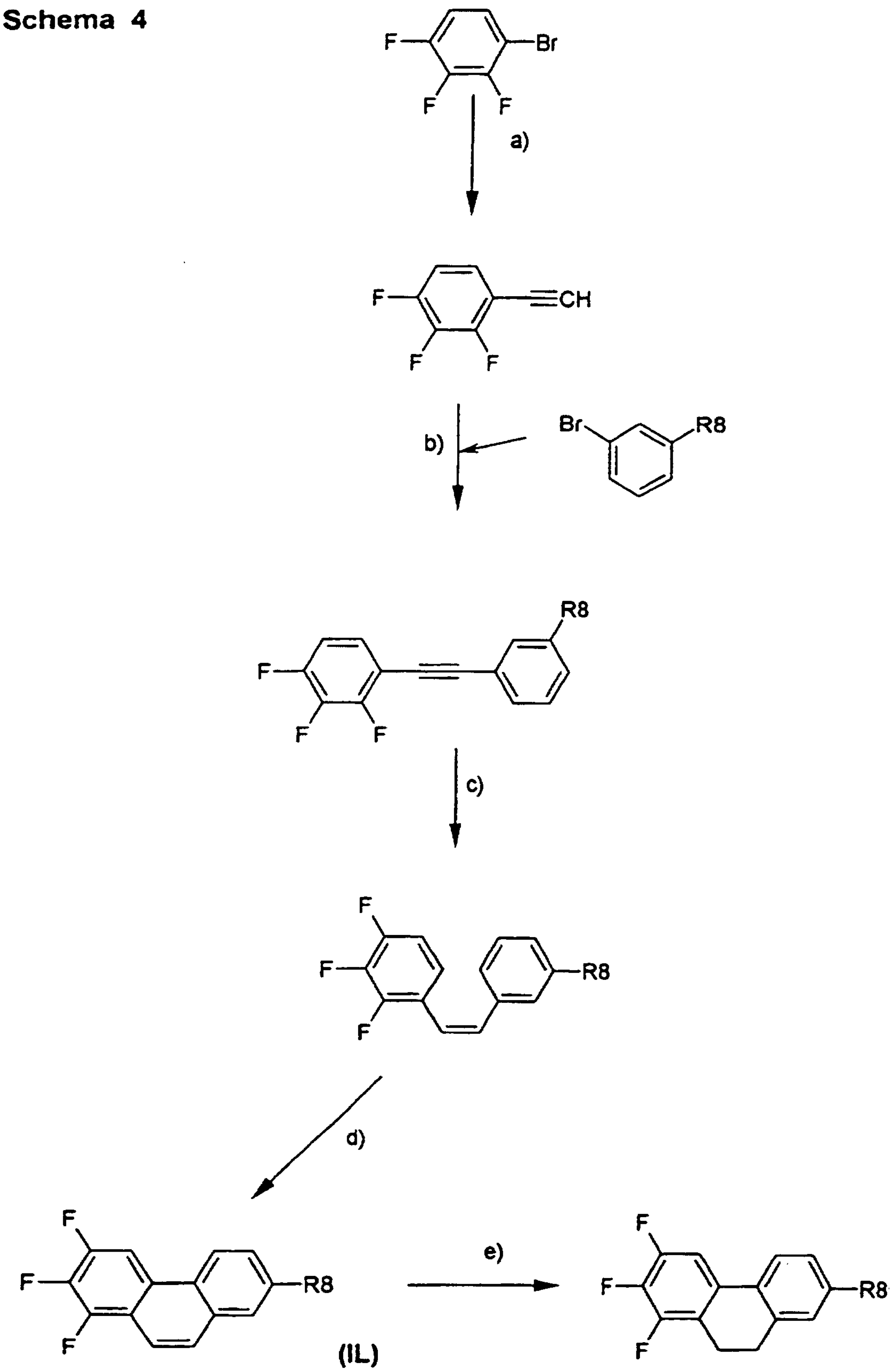


(IL)

(Im)

a) H-C≡C-TMS, $HN(iPr)_2$, Pd(II) / Cu(I)-Katalysator ; z.B. J.Organomet,Chem.453 (1993) 2, C19

b) $HN(iPr)_2$, Pd(0)-Katalysator; analog J.Organomet.Chem. 453 (1993) 2, C19

c) $H_2$ /Katalysator (P.N.Rylander, Hydrogenation Methods, Academic Press, London, 1985, p.53)

d) Photocyclisierung (z.B. J.Am.Chem.Soc. 84, 4361 (1962))

e) $H_2$ / Pd (C )

$R^8$: entspricht $R^2$ bzw. kann durch geeignete nachgeschaltete Reaktionen in $R^2$ überführt werden

Allgemein sei für die Synthese der Gruppen. (-A'-M'), $R^1$, -$CH_2$-$R^2$ beispielsweise verwiesen auf DE-A 23 44 732, 24 50 088, 24 29 093, 25 02 94, 26 36 684, 27 01 591 und 27 52 975 für Verbindungen mit 1,4-Cyclohexylen und 1,4-Phenylen-Gruppen.

Dioxanderivate werden zweckmäßig durch Reaktion eines entsprechenden Aldehyds (oder eines seiner reaktionsfähigen Derivate) mit einem entsprechenden 1 ,3-Diol (oder einem seiner reaktionsfähigen Derivate) hergestellt, vorzugsweise in Gegenwart eines inerten Lösungsmittels, wie Benzol oder Toluol, und/oder eines Katalysators, z.B. einer starken Säure, wie Schwefelsäure, Benzol-oder p-Toluolsulfonsäure, bei Temperaturen zwischen etwa 20°C und etwa 150°C, vorzugsweise zwischen 80°C und 120°C. Als reaktionsfähige Derivate der Ausgangsstoffe eignen sich in erster Linie Acetale.

Die genannten Aldehyde und 1,3-Diole sowie ihre reaktionsfähigen Derivate sind zum Teil bekannt, zum Teil können sie ohne Schwierigkeiten nach Standardverfahren der Organischen Chemie aus literaturbekannten Verbindungen hergestellt werden. Beispielsweise sind die Aldehyde durch Oxydation entsprechender Alkohole oder durch Reduktion von Nitrilen oder entsprechenden Carbonsäuren oder ihrer Derivate, die Diole durch Reduktion entsprechender Diester erhältlich.

Verbindungen, worin ein aromatischer Ring durch mindestens ein F-Atom substituiert ist, können auch aus den entsprechenden Diazoniumsalzen durch Austausch der Diazoniumgruppe gegen ein Fluoratom, z.B. nach den Methoden von Balz und Schiemann, erhalten werden.

Was die Verknüpfung der Ringsysteme miteinander angeht, sei beispielsweise verwiesen auf:
N. Miyaura, T. Yanagai und A. Suzuki in Synthetic Communications 11 (1981), 513-519, DE-C 39 30 663, M.J. Sharp, W. Cheng, V. Snieckus in Tetrahedron Letters 28 (1987) 5093; G.W. Gray in J. Chem. Soc. Perkin Trans II 1989, 2041 und Mol. Cryst. Liq. Cryst. 172 (1989) 165, 204 (1991) 43 und 91; EP-A 0 449 015; WO-A 89/12039; WO-A 89/03821; EP-A 0 354 434 und EP-A 0 694 530 für die direkte Verknüpfung von Aromaten und Heteroaromaten; DE-A 32 01 721 für Verbindungen mit -$CH_2CH_2$-Brückengliedern und Koji Seto et al. in Liquid Crystals 8 (1990) 861-870 für Verbindungen mit -C≡C-Brückengliedern.

Ester der Formel (I) können auch durch Veresterung entsprechender Carbonsäuren (oder ihrer reaktionsfähigen Derivate) mit Alkoholen bzw. Phenolen (oder ihren reaktionsfähigen Derivaten), nach der DCC-Methode (DCC = Dicyclohexylcarbodiimid) oder analog DE-A 44 27 198 erhalten werden.
Die entsprechenden Carbonsäuren und Alkohole bzw. Phenole sind bekannt oder können in Analogie zu bekannten Verfahren hergestellt werden.

Als reaktionsfähige Derivate der genannten Carbonsäuren eignen sich insbesondere die Säurehalogenide, vor allem die Chloride und Bromide, ferner die Anhydride, z.B. auch gemischte Anhydride, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls, wie Natrium oder Kalium, in Betracht.
Die Veresterung wird vorteilhaft in Gegenwart eines inerten Lösungsmittels durchgeführt. Gut geeignet sind insbesondere Ether, wie Diethylether, Di-nbutylether, THF, Dioxan oder Anisol, Ketone, wie Aceton, Butanon oder Cyclohexanon, Amide, wie DMF oder Phosphorsäurehexamethyltriamid, Kohlenwasserstoffe, wie Benzol, Toluol oder Xylol, Halogenkohlenwasserstoffe, wie Tetrachlorkohlenstoff, Dichlormethan oder Tetrachlorethylen und Sulfoxide, wie Dimethylsulfoxid oder Sulfolan.

Ether der Formel (I) sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, Alkylsulfonat oder Dialkylsulfat umgesetzt werden, zweckmäßig in einem inerten Lösungsmittel, wie Aceton, 1,2-Dimethoxyethan, DMF oder Dimethylsulfoxid, oder auch mit einem Überschuß an wäßriger oder wäßrig-alkoholischer NaOH oder KOH bei Temperaturen zwischen etwa 20° und 100°C.

Was die Synthese spezieller Reste $R^1$ und $R^2$ angeht, sei zusätzlich beispielsweise verwiesen auf
EP-A 0 355 008 für Verbindungen mit siliziumhaltigen Seitenketten,
EP-A 0 292 954 für optisch aktive Verbindungen mit Oxiranestereinheit,
EP-A 0 263 437 für optisch aktive Verbindungen mit Oxiranethereinheit,
EP-A 0 361 272 für optisch aktive Verbindungen mit Dioxolanestereinheit,
EP-A 0 351 746 für optisch aktive Verbindungen mit Dioxolanethereinheit,
US 5,051,506 für optisch aktive Verbindungen mit 2,3-Difluoralkyloxy-Einheit,
US 4,798,680 für optisch aktive Verbindungen mit 2-Fluoralkyloxy-Einheit,
US 4,855,429 für optisch aktive Verbindungen mit $\alpha$-Chlorcarbonsäure-Einheit,
EP-A 0 552 658 für Verbindungen mit Cyclohexylpropionsäureresten,
EP-A 0 318 423 für Verbindungen mit Cyclopropylgruppen in der Seitenkette.

Gegenstand der Erfindung ist auch die Verwendung von Verbindungen der Formel (I) in Flüssigkristallmischungen, vorzugsweise smektischen und nematischen, besonders bevorzugt ferroelektrischen und nematischen. Insbesondere bevorzugt ist die Verwendung in ferroelektrischen Flüssigkristallmischungen, die im Inverse-Mode betrieben werden sowie in nematischen Flüssigkristallmischungen, für "active matrix", super TFT-IPS- und/oder ECB-Anwendungen.

Weiterhin Gegenstand der Erfindung sind Flüssigkristallmischungen, vorzugsweise smektische und nematische, besonders bevorzugt ferroelektrische und nematische, enthaltend eine oder mehrere Verbindungen der Formel (I).
Die erfindungsgemäßen smektischen oder nematischen Flüssigkristallmischungen eignen sich vorzugsweise für den Einsatz in elektrooptischen Displays, im Falle von nematischen Mischungen besonders für "Active Matrix Displays" und "In-plane-switching Displays" (IPS-LCD), im Falle von smektischen Flüssigkristallmischungen für ECB-Displays (Electrically Controlled Birefringence), für elektrokline Displays und chirale geneigt smektische (ferroelektrische oder antiferroelektrische) Displays.

Die erfindungsgemäßen Flüssigkristallmischungen enthalten im allgemeinen 2 bis 35, vorzugsweise 2 bis 25, besonders bevorzugt 2 bis 20 Komponenten.

Sie enthalten im allgemeinen 0,01 bis 80 Gew.-%, vorzugsweise 0,1 bis 60 Gew.-%, besonders bevorzugt 0,1 bis 30 Gew.-%, an einer oder mehreren, vorzugsweise 1 bis 10, besonders bevorzugt 1 bis 5, ganz besonders bevorzugt 1 bis 3, der erfindungsgemäßen Verbindungen der Formel (I).

Weitere Komponenten von Flüssigkristallmischungen, die erfindungsgemäße Verbindungen der Formel (I) enthalten, werden vorzugsweise ausgewählt aus den bekannten Verbindungen mit smektischen und/oder nematischen und/oder cholesterischen Phasen. Dazu gehören z. B.:

- Derivate des Phenylpyrimidins, wie beispielsweise in WO-A 86/06401 und US-4 874 542 beschrieben,
- metasubstituierte Sechsringaromaten, wie beispielsweise in EP-A 0 578 054 beschrieben,
- Siliziumverbindungen, wie beispielsweise in EP-A 0 355 008 beschrieben,
- mesogene Verbindungen mit nur einer Seitenkette, wie beispielsweise in EP-A 0 541 081 beschrieben,
- Hydrochinonderivate, wie beispielsweise in EP-A 0 603 786 beschrieben,
- Phenylbenzoate, wie beispielsweise bei P. Keller, Ferroelectrics 1984, 58, 3 und J. W. Goodby et al., Liquid Crystals and Ordered Fluids, Bd. 4, New York 1984 beschrieben, und
- Thiadiazole, wie beispielsweise in EP-A 0 309 514 beschrieben.

Als chirale, nicht racemische Dotierstoffe kommen beispielsweise in Frage:

- optisch aktive Phenylbenzoate, wie beispielsweise bei P. Keller, Ferroelectrics 1984, 58, 3 und J. W. Goodby et al., Liquid Crystals and Ordered Fluids, Bd. 4, New York 1984 beschrieben,
- optisch aktive Oxiranether, wie beispielsweise in EP-A 0 263 437 und WO-A 93/13093 beschrieben,
- optisch aktive Oxiranester, wie beispielsweise in EP-A 0 292 954 beschrieben,
- optisch aktive Dioxolanether, wie beispielsweise in EP-A 0 351 746 beschrieben,
- optisch aktive Dioxolanester, wie beispielsweise in EP-A 0 361 272 beschrieben,
- optisch aktive Tetrahydrofuran-2-carbonsäureester, wie beispielsweise in EP-A 0 355 561 beschrieben, und

- optisch aktive 2-Fluoralkylether, wie beispielsweise in EP-A 0 237 007, EP-A 0 428 720 und US-5,051,506 beschrieben.

Bevorzugte weitere Komponenten von FLC-Mischungen, die im Inverse-Mode eingesetzt werden, sind:

Phenanthrenderivate der Formel (II),

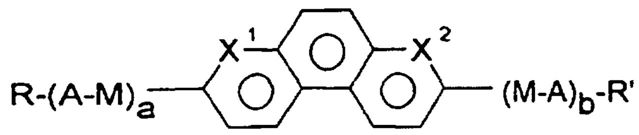

( II )

Fluorpyridine der Formel (III),

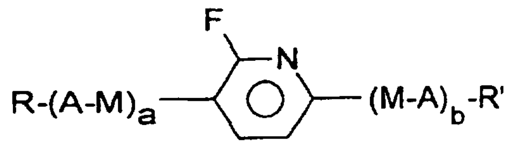

( III )

Difluorphenylenderivate der Formel (IV)

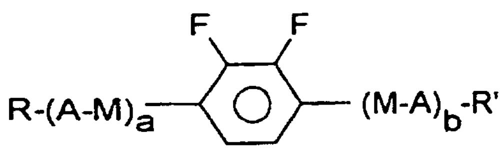

( IV )

Metasubstituierte aromatische Verbindungen der Formel (V)

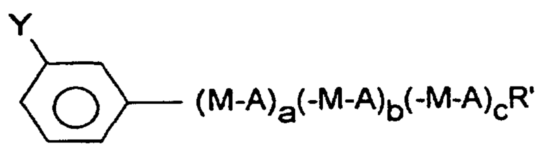

( V )

4-Cyanocyclohexyle der Formel (VI)

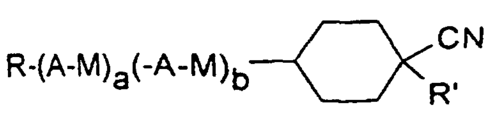

( VI )

1,3,4-Thiadiazole der Formel (VII),

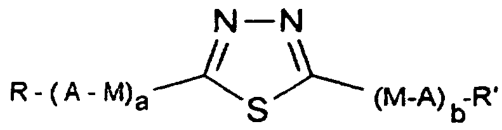

(VII)

wobei die Symbole und Indizes die folgenden Bedeutungen haben:

$X^1$, $X^2$ sind gleich oder verschieden unabhängig voneinander CH, CF oder N;
Y ist F, $CF_3$ oder R;
R, R' haben, gleich oder verschieden, unabhängig voneinander die gleichen Bedeutungen wie $R^1$, $R^2$ in Formel (I);
A, M haben, gleich oder verschieden, unabhängig voneinander die gleichen Bedeutungen wie in Formel (I) und
a,b;c,d sind gleich, oder verschieden, unabhängig voneinander 0 oder 1, mit der Maßgabe, daß die Verbindungen nicht mehr als vier Ringsysteme enthalten dürfen und, mit Ausnahme der Formel (II), mindestens zwei Ringsysteme enthalten

müssen.

Geeignete weitere Bestandteile erfindungsgemäßer nematischer bzw. chiral nematischer Flüssigkristallmischungen sind beispielweise

- 4-Fluorbenzole, wie beispielsweise in EP-A 494 368, WO 92/06 148, EP-A 460 436, DE-A 4 111 766, DE-A 4 112 024, DE-A 4 112 001, DE-A 4 100 288, DE-A 4 101 468, EP-A 423 520, DE-A 392 3064, EP-A 406 468, EP-A 393 577, EP-A 393 490 beschrieben,
- 3,4-Difluorbenzole, wie beispielsweise in DE-A 4 108 448, EP-A 507 094 und EP-A 502 407 beschrieben,
- 3,4,5-Trifluorbenzole, wie beispielsweise in DE-A 4 108 448, EP-A 387 032 beschrieben,
- 4-Benzotrifluoride, wie beispielsweise in DE-A 4 108 448 beschrieben,
- Phenylcyclohexane, wie beispielsweise in DE-A 4 108 448 beschrieben.

Die Mischungen wiederum können Anwendung finden in elektrooptischen oder vollständig optischen Elementen, z. B. Anzeigeelementen, Schaltelementen, Lichtmodulatoren, Elementen zur Bildbearbeitung und/oder Signalverarbeitung oder allgemein im Bereich der nichtlinearen Optik.

Ferner sind die Mischungen für Feldbehandlung, d. h. zum Betrieb in der Quasi-Bookshelf-Geometrie (QBG) (siehe z. B. H. Rieger et al., SID 91 Digest (Anaheim) 1991, 396) geeignet.

Die erfindungsgemäßen ferroelektrischen Flüssigkristallmischungen eignen sich insbesondere zum Betrieb im sogenannten Inverse- oder $V_{(min)}$-Mode (siehe z. B.: J. C. Jones, M. J. Towler, J. R; Hughes, Displays 1993,14, Nr. 2, 86-93; M. Koden, Ferroelectrics 1996, 179, 121-129).

Flüssigkristalline Mischungen, die Verbindungen der allgemeinen Formel (I) enthalten, sind besonders für die Verwendung in elektrooptischen Schalt- und Anzeigevorrichtungen (Displays) geeignet. Diese Displays sind üblicherweise so aufgebaut, daß eine Flüssigkristallschicht beiderseitig von Schichten eingeschlossen ist, die üblicherweise, in dieser Reihenfolge ausgehend von der LC-Schicht, mindestens eine Orientierungsschicht, Elektroden und eine Begrenzungsscheibe (z. B. aus Glas) sind. Darüberhinaus können sie Abstandshalter, Kleberahmen, Polarisatoren sowie für Farbdisplays dünne Farbfilterschichten enthalten. Weitere mögliche Komponenten sind Antireflex-, Passivierungs-, Ausgleichs- und Sperrschichten sowie elektrisch-nichtlineare Elemente, wie Dünnschichttransistoren (TFT) und Metall-Isolator-Metall-(MIM)-Elemente. Im Detail ist der Aufbau von Flüssigkristalldisplays bereits in einschlägigen Monographien beschrieben (siehe z. B. E. Kaneko, "Liquid Crystal TV Displays: Principles and Applications of Liquid Crystal Displays", KTK Scientific Publishers, 1987).

Ein weiterer Gegenstand der Erfindung ist daher eine Schalt- und/oder Anzeigevorrichtung, vorzugsweise eine smektische oder nematische, insbesondere eine ferroelektrische, enthaltend eine Flüssigkristallmischung, die eine oder mehrere Verbindungen der Formel (I) enthält.
Bei Vorichtungen, die eine nematische Flüssigkristallmischung enthalten sind "Active Matrix Displays" und "In-plane-switching Displays" (IPS-LCD) bevorzugt.

Bei Vorrichtungen, die eine smektische Flüssigkristallmischung enthalten, sind ECB-Displays (Electrically Controlled Birefringence), elektrokline Displays und chirale geneigt smektische (ferroelektrische oder antiferroelektrische) Displays bevorzugt.

Solche Vorrichtungen können beispielsweise als Computerdisplays oder in Chipkarten Anwendung finden.

Vorzugsweise wird eine erfindungsgemäße ferroelektrische Schalt- und/oder Anzeigevorrichtung im Normal- oder Inverse-Mode betrieben.

Durch Multiplexansteuerung betriebene ferroelektrische Schalt- und/oder Anzeigevorrichtungen können unter anderem auf zwei unterschiedliche Weisen betrieben werden, die sogenannte normale (Normal-Mode) oder die sogenannte inverse (Inverse Mode auch $V_{(min)}$-Mode). Der Unterschied zwischen beiden liegt im Ansteuerschema und in den verschiedenen Anforderungen an den dielektrischen Tensor des FLC-Materials, d. h. der FLC-Mischung. Einen Überblick geben z. B. J. C. Jones et al. in Displays 1993, 14, Nr. 2, 86-93 im folgenden als "Jones" bezeichnet, und M. Koden in Ferroelectrics 1996, 179, 121-129, sowie die dort aufgeführte Literatur.

Die Schaltcharakteristika einer FLC-Vorrichtung können im allgemeinen durch ein Diagramm dargestellt werden, bei dem die Treiberspannung (V) horizontal und die Breite der Ansteuerpulse ( , Zeit) vertikal aufgetragen sind (siehe z. B. Jones, Abb. 4, 8, 10 und 11).

Eine Schaltkurve wird experimentell bestimmt und teilt die V, -Fläche in einen schaltenden - und einen nicht schaltenden Bereich. Üblicherweise verkürzt sich bei Erhöhung der Spannung die Pulsbreite. Dieses Verhalten kennzeichnet den sogenannten Normal-Mode (siehe z. B. Jones, Abb. 4).

Bei geeigneten Materialien weist die V -Kurve jedoch ein Minimum auf (bei der Spannung $V_{(min)}$, wie z. B. bei Jones in den Abbildungen 8, 10 und 11 zu sehen. Dieses Minimum kommt:durch Überlagerung von dielektrischer und ferroelektrischer . Verdrillung zustande. FLC-Vorrichtungen werden im Inverse-Mode betrieben, wenn die Summe der Zeilen- und Spalten-Treiberspannung im Arbeitstemperaturbereich höher als das Minimum auf der V -Kurve sind, d. h. $V_{(zeile)} + V_{(Spalte)} > V_{(min)}$.

Ethin-Derivaten der Formel

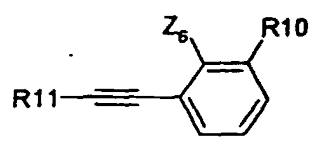

worin bedeuten:

R10 einen Alkyl- oder Alkyloxy-Rest mit 1 bis 16 C-Atomen

R11 H, wenn $Z_6$ F bedeutet ; oder

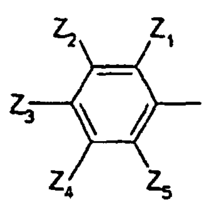

worin bedeuten:

a) $Z_1$ : F; $Z_2$: Alkyl- oder Alkyloxy mit 1 bis 16 C-Atomen ; $Z_3$, $Z_4$, $Z_5$: H; $Z_6$ : F
b) $Z_1$, $Z_2$ , $Z_3$ : F; $Z_4$, $Z_5$ : H; $Z_6$: F
c) $Z_1$, $Z_3$ : F; $Z_2$: H ; $Z_4$, $Z_5$ : H ; $Z_6$: F
d) $Z_1$, $Z_3$ : F; $Z_2$: H ; $Z_4$, $Z_5$ : H ; $Z_6$ : H
e) $Z_2$, $Z_3$ : F; $Z_1$: H ; $Z_4$, $Z_5$ : H; $Z_6$: H
f) $Z_1$, $Z_2$ , $Z_3$ : F; $Z_4$, $Z_5$ : H; $Z_6$: H
g) $Z_1$: H; $Z_2$ : Cl ; $Z_3$ : F; $Z_4$, $Z_5$ : H; $Z_6$: H

kommt in dem bevorzugten Verfahren zur Herstellung der Verbindungen-der Formel (I) eine Schlüsselfunktion zu, da sie einerseits mit sehr guten Ausbeuten und hohen Reinheiten ohne-Anfall größerer.Mengen an Nebenprodukten zu den entsprechenden Ethen-Derivaten (die ihrerseit photocyclisiert werden; s.a. Schemata 1-7) hydriert werden können, andererseits in einer konvergenten, Homologisierung leicht ermöglichenden Reaktion mit hohen Ausbeuten und sehr

guten Reinheiten zugänglich sind.
Es wurde gefunden, daß diese Reaktionsabfolge *1-Aryl-2-aryl'-ethin - 1-Aryl-2-aryl'-ethen - Photocyclisierung* nicht nur für die erfindungsgemäßen Phenanthren-Derivate in besonderem Maße geeignet ist, sondern sich auch in besonderem Maße zur Herstellung von Phenanthren-Derivate mit anderem Substitutionsmuster elektronegativer Reste (wie z.B. in der US 5,648,021 oder DE-A 19524230 mit dort noch anderen Herstellverfahren beschrieben) eignet.

Die Erfindung wird.durch das nachfolgende Beispiel weiter erläutert, ohne.sie dadurch beschränken zu wollen.

**Beispiel 1: 2-Hexyloxy-1,6,7,8-tetrafluor-phenanthren**

Eine Lösung von 112 g 2,6-Dibrom-fluorbenzol [1435-54-7] in 1000 ml Diethylether wird bei -70°C tropfenweise mit 290 ml einer 1.6 molaren Lösung von Butyllithium in Hexan versetzt. Nach 2 h bei gleicher Temperatur wird mit 60 ml Trimethylborat versetzt und für 12 h bei dieser Temperatur gerührt. Nach langsamen Erreichen der Raumtemperatur wird mit 300 ml Wasser versetzt und durch Zugabe von Chlorwasserstoffsäure pH 1 eingestellt. Die organische Phase wird abgetrennt, die wäßrige zweimal mit je 100 ml Ether extrahiert und die vereinigten organischen Phasen im Vakuum zur Trockne gebracht. Es resultieren 91 g 3-Brom-2-fluorphenylboronsäure (gemischt mit ihrem cyclischen Anhydrid) als braunerFeststoff; $^1$H-NMR (DMSO-$d_6$) : 7.1-7.7 (m) ppm $^{19}$F-NMR (DMSO-$d_6$ ) : -99 ppm .
Die rohe 2-Brom-1-fluor-phenylboronsäure. (80 g) wird, in 300 ml tert-Butylmethylether gelöst, bei Raumtemperatur tropfenweise mit 125 ml einer 35%igen wäßrigen Lösung von Wasserstoffperoxid versetzt; dabei steigt die Temperatur bis auf 55 °C. Nach beendeter Zugabe wird noch 2 h zum Rückfluß erhitzt. Nach dem Abkühlen werden 100 ml Wasser zugegeben und die organische Phase zweimal mit gesättigter Natriumsulfitlösung ausgeschüttelt. Nach Abdestillation des Lösungsmittel im Vakuum wird durch Vakuumdestillation (90°C; 4mbar) 54g 3-Brom-2-fluor-phenol als bald kristallisierendes Öl erhalten.
$^1$H-NMR ($CDCl_3$ ) : 6.9-7.1 (m; 3H); 5.1 (br; 1 H) $^{19}$F-NMR ($CDCl_3$) : -135 ppm. Eine Lösung von 53,6 g 3-Brom-2-fluorphenol in 650 ml Aceton wird mit 117 g Kaliumcarbonat sowie 55.6 g Bromhexan versetzt und für 8 h zum Rückfluß erhitzt. Nach Filtration wird auf 1/3 des ursprünglicxhen Volumens eingedampft und in 600 ml Wasser gegossen. Mit Chlorwasserstoffsäure wird pH1 eingestellt und dreimal mit je 150 ml tert-Butylmethylether extrahiert. Die vereinigten organischen Phasen werden im Vakuum konzentriert und der Rückstand einer Vakuumdestillation (103 °C; 1 mbar) unterzogen; es resultieren 67 g 6-Brom-2-hexyloxy-fluorbenzol als farbloses Öl. $^1$H-NMR ($CDCl_3$ ) : 6.9-7.1 (m; 3H); 4.0 (t; 2H) 0.9-1.8 (m, 11 H)
$^{19}$F-NMR ($CDCl_3$ ) : -128 ppm.
Eine Lösung von 67.3 g 6-Brom-2-hexyloxy-fluorbenzol, 24.7 g 2-Methyl-3-butin-2-ol [115-19-5], 0.7 g bis(Triphenylphosphin)palladium(II)chlorid, und 0.7 g Kupferiodid in 340 ml Triethylamin wird 5 h zum Rückfluß erhitzt. Anschließend wird auf 1.5 l Eiswasser gegossen, mit Chlorwasserstoffsäure pH1 eingestellt und dreimal mit je 200 ml tert-Butylmethylether extrahiert. Die vereinigten organischen Phasen werden im Vakuum zur Trockne gebracht. Das rohe 4-(2-Fluor-3-hexyloxyphenyl)-2-methyl-3-butin-1-ol wird mit drei Mol-Äquivanten Natriumhydroxid für 3 h in 1000 ml Toluol zum Rückfluß erhitzt. Nach Filtration wird verdünnter Chlorwasserstoffsäure gewaschen, das Lösungsmittel abdestilliert und der Rückstand destilliert (90°C; 0.5 mbar) ; es resultieren 34 g 1-(2-Fluor-3-hexyloxyphenyl)ethin.
$^1$H-NMR ($CDCl_3$ ) : 7.0 (m; 3H); 4.0 (t; 2H); 3.3 ("s", 1 H) 0.9-1.8 (m, 11 H) $^{19}$F-NMR ($CDCl_3$): -132 ppm.

Dessen Umsetzung mit 2,3,4-Trifluorbrombenzol zu 1-(2-Fluor-3-hexyloxyphenyl)-2-(2,3,4-trifluorphenyl) ethin , gefolgt von dessen Hydrierung zu 1-(2-Fluor-3-hexyloxyphenyl)-2-(2,3,4-trifluorphenyl)ethen und Photocyclisierung geschieht analog Beispiel 1.

## Patentansprüche

1. Fluoriertes Phenanthren-Derivat der Formel (I),

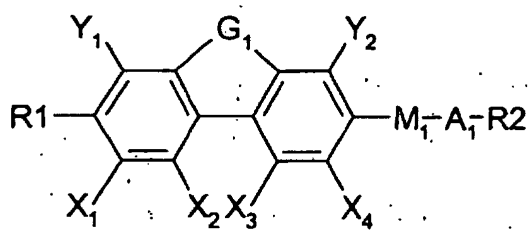

**(I)**

wobei die Symbole und Indizes folgende Bedeutungen haben:

G1 : -CH=CH- oder $-CH_2CH_2-$

$X_1$,$X_2$,$X_3$,$X_4$ : unabhängig voneinander H oder F , mit den Maßgaben, daß

a) $X_1$ und $X_2$ und $X_3$ und $X_4$ nicht zugleich H bedeuten
b) $X_1$ und $X_2$ und $X_3$ und $X_4$ nicht zugleich F bedeuten
c) mindestens ein X aus dieser Gruppe F bedeutet;

$Y_2$ : H oder F; $Y^1$ : F;

$R^1$, $R^2$ sind gleich oder verschieden

a) ein geradkettiger oder verzweigter Alkylrest (mit oder ohne asymmetrisches C-Atom) mit 1 bis 20 C-Atomen, worin auch ein oder mehrere H-Atome durch F substituiert sein kann und wobei

a1) eine oder mehrere nicht benachbarte und nicht terminale $CH_2$-Gruppen durch -O-, -S-, -CO-O-, -O-CO-, -O-CO-O- oder $-Si(CH_3)_2-$ ersetzt sein können und/oder
a2) eine oder mehrere $CH_2$-Gruppen durch -CH=CH-, -C≡C-, Cyclopropan-1,2-diyl, 1,4-Phenylen, 1,4-Cyclohexylen oder 1,3-Cyclopentylen ersetzt sein können und/oder
a3) die terminale $CH_3$-Gruppe durch eine der folgenden chiralen Gruppen (optisch aktiv oder racemisch) ersetzt sein kann:

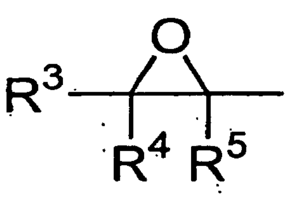

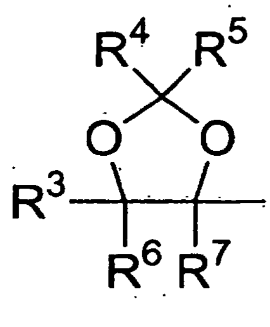

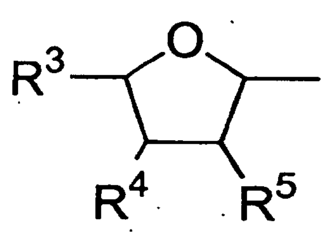

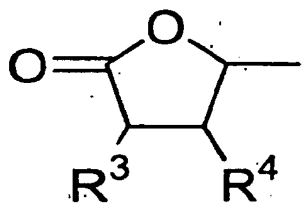

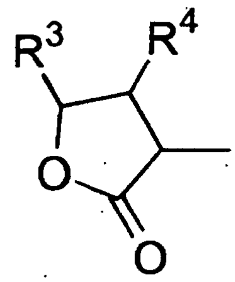

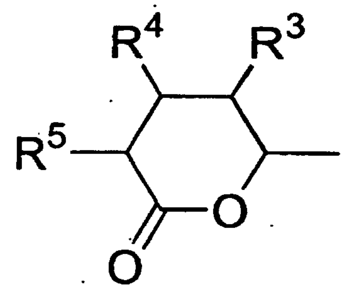

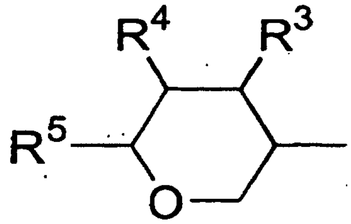

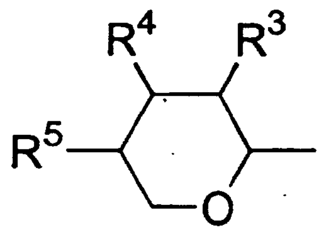

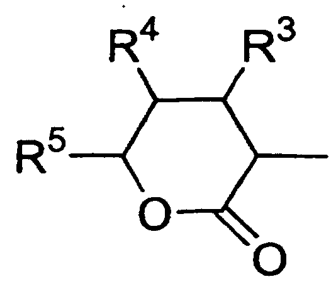

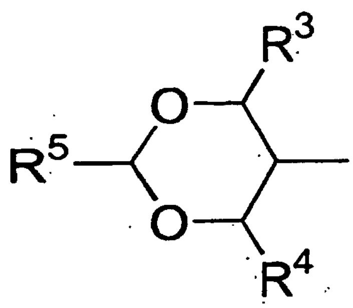

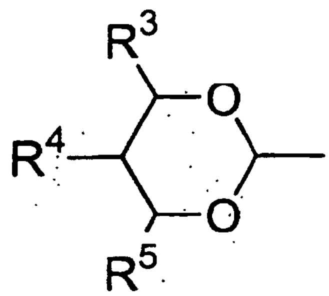

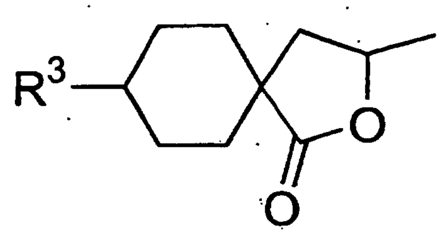

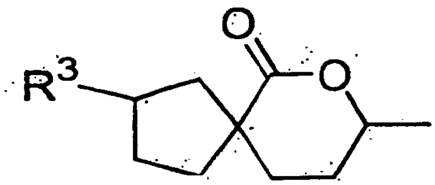

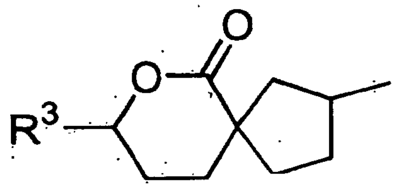

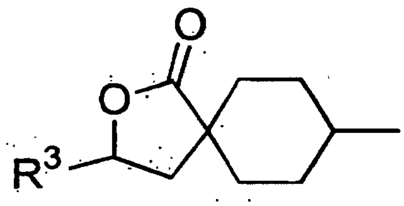

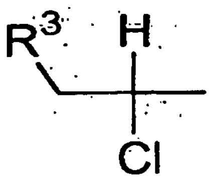

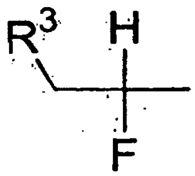

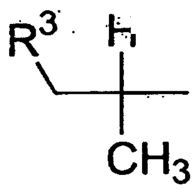

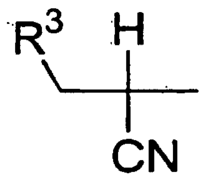

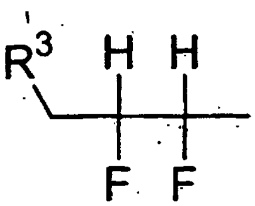

$R^1$ auch Wasserstoff, $-OCF_3$, $-CF_3$, -CN, -F, -Cl, $-OCHF_2$, $-OCH_2F$, $-CHF_2$ oder $-CH_2F$;

b) $R^1$ auch Wasserstoff, Cl oder F

$R^3$, $R^4$, $R^5$, $R^6$, $R^7$ sind gleich oder verschieden

a) Wasserstoff
b) ein geradkettiger oder verzweigter Alkylrest (mit oder ohne asymmetrisches Kohlenstoffatom) mit 1 bis 16 C-Atomen, wobei

b1) eine oder mehrere nicht benachbarte und nicht terminale $CH_2$-Gruppen durch -O- ersetzt sein können und/oder
b2) eine oder zwei $CH_2$-Gruppen durch -CH=CH- ersetzt sein können,

c) $R^4$ und $R^5$ zusammen auch $-(CH_2)_4$-oder $-(CH_2)_5$-, wenn sie an ein Oxiran-, Dioxolan-, Tetrahydrofuran-, Tetrahydropyran-, Butyrolactonoder Valerolacton-System gebunden sind;

mit der Maßgabe, daß $R^3$ nur dann Wasserstoff sein kann, wenn $R^3$ Substituent eines der aufgeführten Ringsysteme ist;

$M^1$ -CO-O-, $-CH_2-O-$, -CH=CH-, -C≡C-, $-CH_2-CH_2-CO-O-$, $-CH_2CH_2CH_2CH_2-$ , $CH_2CH_2CH_2O-$ und deren spiegelbildliche Anordnungen; oder eine Einfachbindung;

$A^1$ 1,4-Phenylen, wobei ein oder mehrere H-Atome durch F, Cl und/oder CN ersetzt sein können, 1,4-Cyclohexylen, wobei ein oder zwei H-Atome durch CN und/oder $CH_3$ und/oder F ersetzt sein können, Naphthalin-2,6-diyl,

wobei ein oder mehrere H-Atome durch F, Cl und/oder CN ersetzt sein können ; oder eine Einfachbindung.

**2.** Fluoriertes Phenanthren-Derivat nach Anspruch 1, wobei die Symbole und Indizes folgende Bedeutungen haben:

G1 : -CH=CH- oder $-CH_2CH_2-$

$X_2$,$X_3$,$X_4$ : unabhängig voneinander H oder F ,

$Y_2$ : H oder F;

$R^1$, $R^2$ sind gleich oder verschieden

a) ein geradkettiger oder verzweigter Alkylrest (mit oder ohne asymmetrisches C-Atom) mit 1 bis 20 C-Atomen, worin auch ein oder mehrere H-Atome durch F substituiert sein kann und wobei

a1) eine öder mehrere nicht benachbarte und nicht terminale $CH_2$-Gruppen durch -O-, -S-, -CO-O-, -O-CO-, -O-CO-O- oder $-Si(CH_3)_2-$ ersetzt sein können und/oder
a2) eine oder mehrere $CH_2$-Gruppen durch -CH=CH-, -C≡C-, Cyclopropan-1,2-diyl, 1,4-Phenylen, 1,4-Cyclohexylen oder 1,3-Cyclopentylen ersetzt sein können und/oder
a3) die terminale $CH_3$-Gruppe durch eine der folgenden chiralen Gruppen (optisch aktiv oder racemisch) ersetzt sein kann:

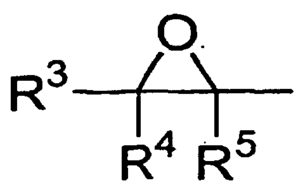

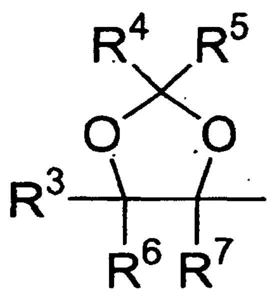

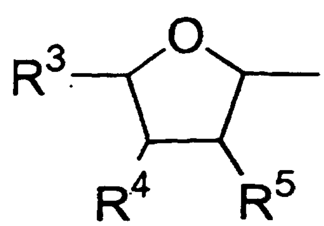

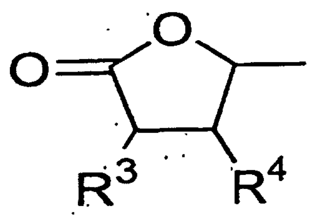

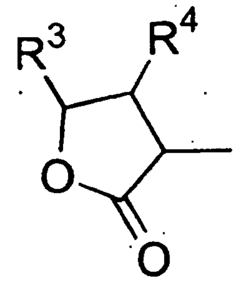

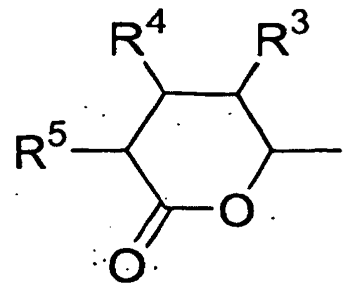

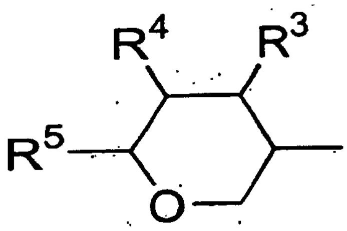

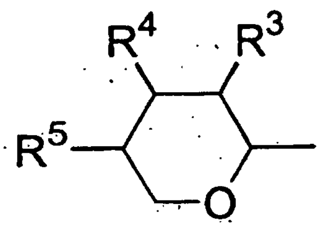

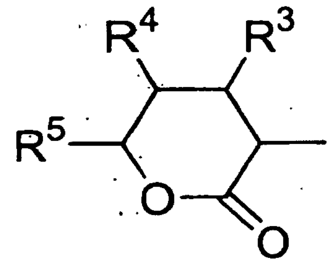

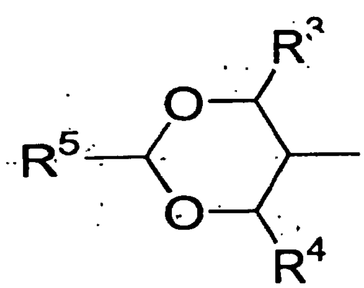

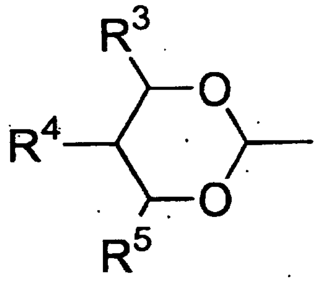

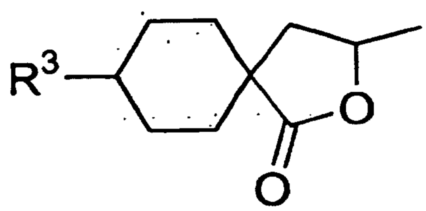

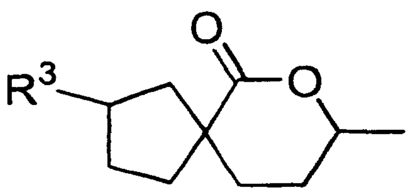
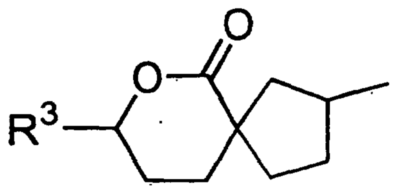
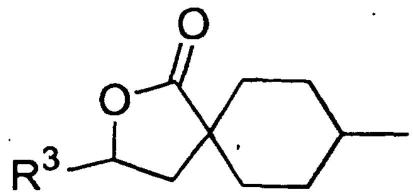

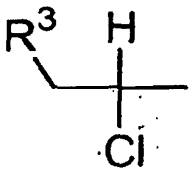
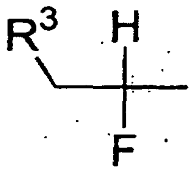
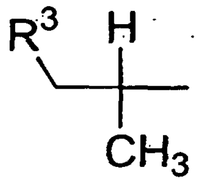
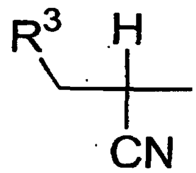

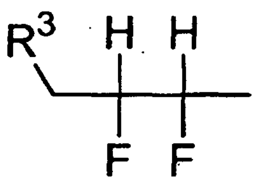

$R^1$ auch Wasserstoff, $-OCF_3$, $-CF_3$, -CN; -F, -Cl, $-OCHF_2$, $-OCH_2F$, $-CHF_2$ oder $-CH_2F$;

b) $R^1$ auch Wasserstoff, Cl oder F

$R^3$, $R^4$, $R^5$, $R^6$, $R^7$ sind gleich oder verschieden

a) Wasserstoff

b) ein geradkettiger oder verzweigter Alkylrest (mit oder ohne asymmetrisches Kohlenstoffatom) mit 1 bis 16 C-Atomen, wobei

b1) eine oder mehrere nicht benachbarte und nicht terminale $CH_2$-Gruppen durch -O- ersetzt sein können und/oder

b2) eine oder zwei $CH_2$-Gruppen durch -CH=CH- ersetzt sein können,

c) $R^4$ und $R^5$ zusammen auch $-(CH_2)_4-$ oder $-(CH_2)_5-$ , wenn sie an ein Oxiran-, Dioxolan-, Tetrahydrofuran-, Tetrahydropyran-,.Butyrolactonoder Valerolacton-System gebunden sind;

mit der Maßgabe, daß $R^3$ nur dann Wasserstoff sein kann, wenn $R^3$ Substituent eines der aufgeführten Ringsysteme ist;

$M^1$ eine Einfachbindung;

$A^1$ eine Einfachbindung.

$X_1$, $Y_1$, Fluor

mit den Maßgaben, dass

i) $Y_2$, $X_2$, $X_3$, $X_4$ Wasserstoff sind, oder
ii) $Y_2$, $X_2$ Fluor und $X_3$, $X_4$ Wasserstoff sind, oder
iii) $Y_2$, $R_1$ Fluor und $X_2$, $X_3$, $X_4$ Wasserstoff sind, oder
iiii) $X_2$ Fluor ist und $Y_2$, $X_3$, $X_4$ Wasserstoff sind, $G_1$-CH = CH-ist, $R_1$ H, Cl, F, $CF_3O$, $CH_2HO$, $CF_3$, ein geradkettiger Alkylrest mit 1 bis 5 C-Atomen und $R_2$ ein geradkettiger Alkyl- oder Alkyloxyrest mit 1 bis 5 C-Atomen ist.

**3.** Flüssigkristallmischung, enthaltend ein oder mehrere Verbindungen der Formel (I) gemäß einem der Ansprüche 1 oder 2.

**4.** Flüssigkristallmischung gemäß Anspruch 3, **dadurch gekennzeichnet, dass** sie chiral geneigt smektisch oder nematisch ist.

**5.** Flüssigkristallmischung gemäß Anspruch 4., **dadurch gekennzeichnet, dass** sie 0,01 bis 80 Gew.-% an einem oder mehreren Derivaten der Formel (I) enthält.

**6.** Schalt- oder Anzeigevorrichtung, enthaltend eine Flüssigkristallmischung gemäß Anspruch 5.

**7.** Schaft- und Anzeigevorrichtung in Form

- einer ferroelektrischen Schalt- und/oder Anzeigevorrichtung, enthaltend eine chiral geneigt smektische Flüssigkristallmischung nach Anspruch 4, oder
- einer Active Matrix-Anzeigevorrichtung, enthaltend eine Flüssigkristallmischung nach Anspruch 4, oder
- einer Super-TFT (IPSI)-Anzeigevorrichtung, enthaltend eine nematische Flüssigkristallmischung nach Anspruch 4, oder
- einer ECB-Anzeigevorrichtung, enthaltend eine nematische Flüssigkristallmischung nach Anspruch 4.

**8.** Monostabiles ferroelektrisches Aktivmatrixdisplay, enthaltend eine Flüssigkristallmischung nach Anspruch 4.

## Claims

**1.** A fluorinated phenanthrene derivative of the formula (I)

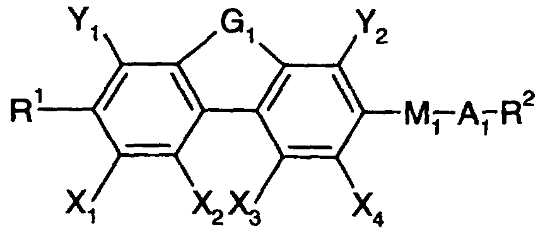

(I)

where the symbols and indices are defined as follows:

| | |
|---|---|
| $G_1$ | is $-CH{=}CH-$ or $-CH_2CH_2-$; |
| $X_1$, $X_2$, $X_3$ and $X_4$, | independently of one another are H of F, with the provisos that<br>a) $X_1$, $X_2$, $X_3$, $X_4$ are not simultaneously H<br>b) $X_1$, $X_2$, $X_3$, $X_4$ are not simultaneously F |
| $Y_2$: | H or F |
| $Y_1$: | F |
| $R^1$ and $R^2$ | are identical or different and are |

a) a straight-chain or branched alkyl radical (with or without an asymmetrical carbon atom) having 1 to 20 carbon atoms, in which, in addition, one or more H atoms may be replaced by F and where

a1) one or more non-adjacent and non-terminal $-CH_2-$ groups may be replaced by -O-, -S-, -CO-O-, -O-CO-, -O-CO-O- or $-Si(CH_3)_2-$, and/or
a2) one or more $-CH_2-$ groups may be replaced by -CH=CH-, -C≡C-, cyclopropanel,2-diyl, 1,4-phenylene, 1,4-cyclohexylene or 1,3-cyclopentylene, and/or
a3) the terminal $-CH_3$ group may be replaced by one of the following chiral groups (optically active: or racemic):

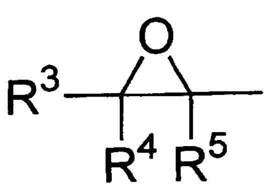

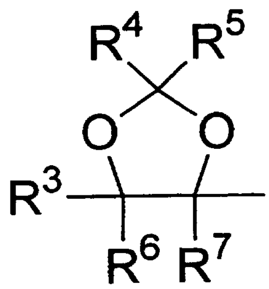

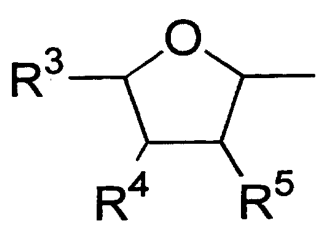

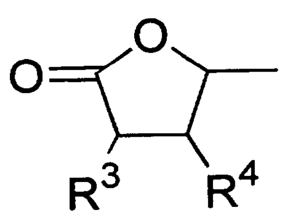

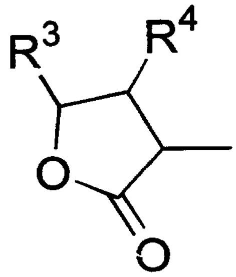

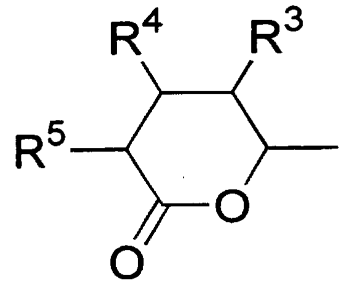

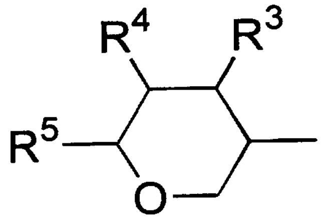

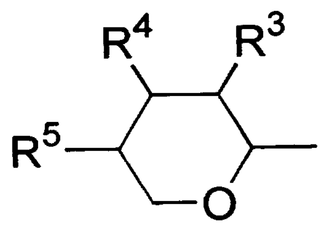

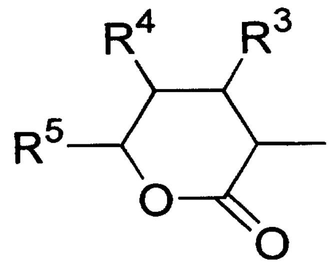

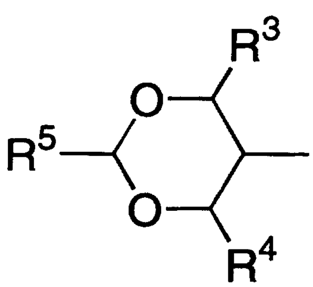

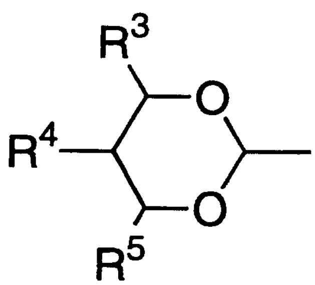

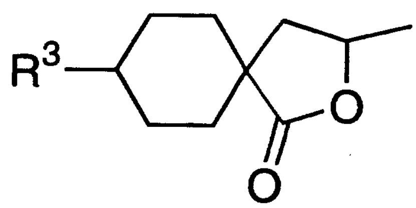

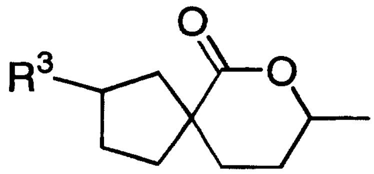

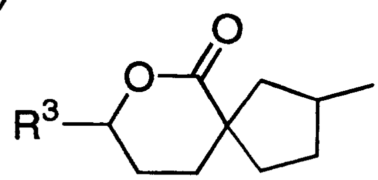

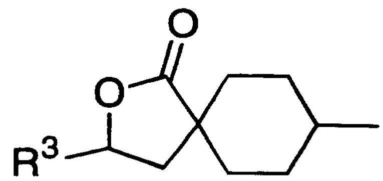

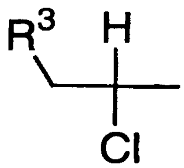

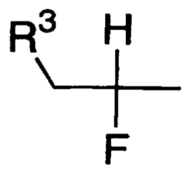

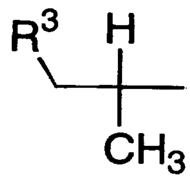

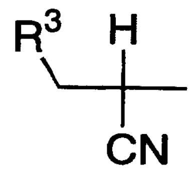

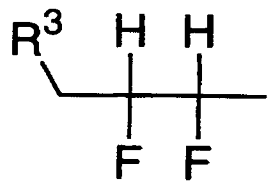

$R^1$ is alternatively hydrogen, $-OCF_3$, $-CF_3$, -CN, -F, -Cl, $-OCHF_2$, $-OCH_2F$, $-CHF_2$ or $-CH_2F$;

b) $R^1$ is alternatively hydrogen, Cl or F;

$R^3$, $R^4$, $R^5$, $R^6$ and $R^7$ are identical or different and are

a) hydrogen
b) a straight-chain or branched alkyl radical (with or without an asymnmetrical carbon atom) having 1 to 16 carbon atoms, where

b1) one or more nonadjacent and non-terminal $-CH_2-$ groups may be replaced by; -O-;
and/or
b2) one or two $-CH_2-$ groups may be replaced by -CH=CH-,

c) $R^4$ and $R^5$ together may alternatively be $-(CH_2)_4-$ or $-(GH_2)_5-$ if they are bonded to an oxirane, dioxolane, tetrahydrofuran, tetrahydropyran, butyrolactone or valerolactone system;

with the proviso that $R^3$ can only be hydrogen If $R^3$ is a substituents of one of the ring systems mentioned;

| | |
|---|---|
| $M^1$ | is -CO-O-, $-CH_2-O-$, -CH=CH-, -C≡C-, $-CH_2-CH_2-CO-O-$, $-CH_2CH_2CH_2CH_2-$, $-CH_2CH_2CH_2O-$ and mirror-image arrangements thereof or a single bond; |
| $A^1$ | is 1,4-phenylene, in which one or more H atoms may be replaced by F, Cl and/or CN, 1,4-cyclohexylene, in which one or two H atoms may be replaced by CN and/or $CH_3$ and/or F, naphthalene-2,6-diyl, in which one or more H atoms may be replaced by F, Cl and/or CN; or a single bond. |

**2.** A fluorinated phenanthrene derivative as claimed in claim 1 where the symbols and indices are defined as follows:

| | |
|---|---|
| $G_1$ | is -CH=CH- or $-CH_2CH_2-$; |
| $X_2$, $X_3$ and $X_4$, | independently of one another. are H of F, |
| $Y_2$: | H or F |
| $R^1$ and $R^2$ | are identical or different and are |

a) a straight-chain or branched alkyl radical (with or without an asymmetrical carbon atom) having 1 to 20 carbon atoms, in which, in addition, one or more H atoms may be replaced by F and where

a1) one or more non-adjacent and non-terminal $-CH_2-$ groups may be replaced by -O-, -S-, -CO-O-, -O-CO-, -O-CO-O- or $-Si(CH_3)_2-$, and/or
a2) one or more $-CH_2-$ groups may be replaced by -CH=CH-, -C≡C-, cyclopropanel,2-diyl, 1,4-phenylene, 1,4-cyclohexylene or 1,3-cyclopentylene, and/or
a3) the terminal $-CH_3$ group may be replaced by one of the following chiral groups (optically active: or racemic):

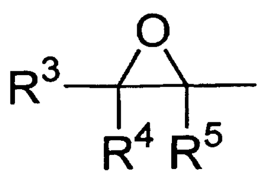
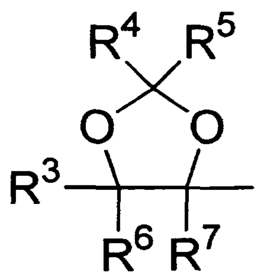
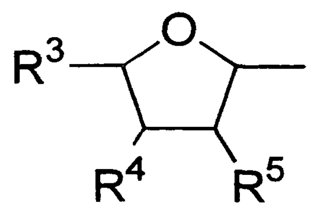

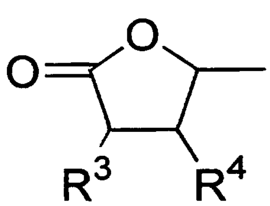
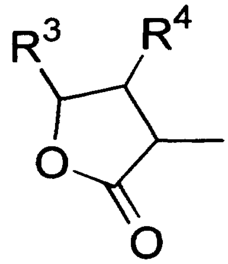
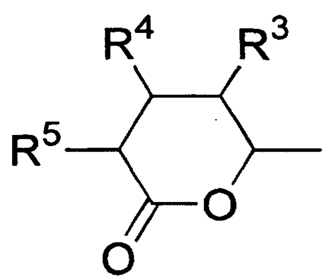

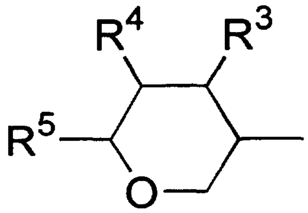
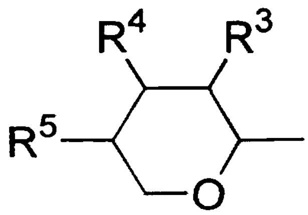
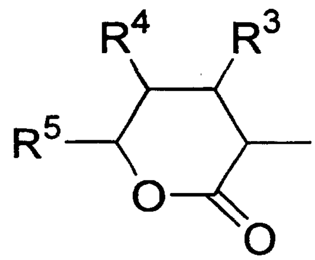

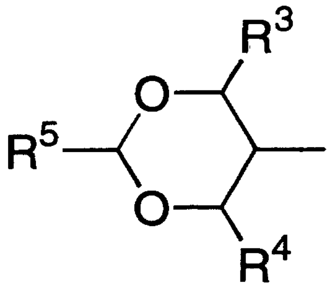
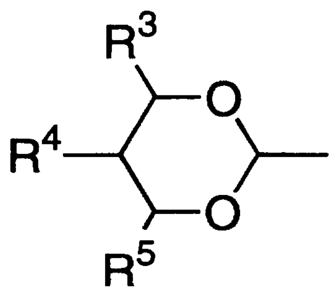
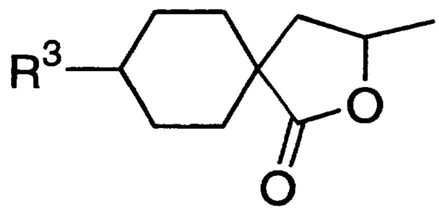

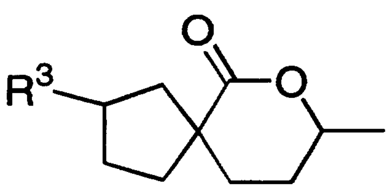
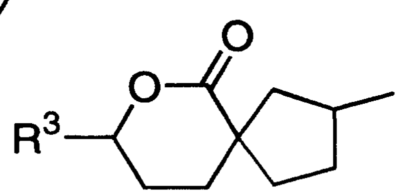
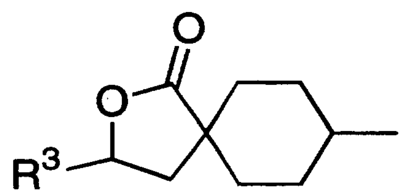

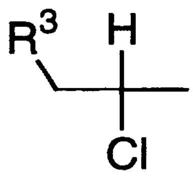 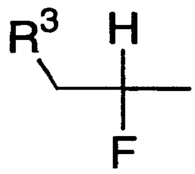 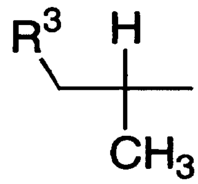 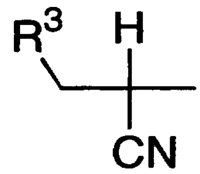

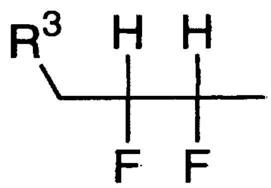

$R^1$ is alternatively hydrogen, $-OCF_3$, $-CF_3$, -CN, -F, -Cl, $-OCHF_2$, $-OCH_2F$, $-CHF_2$ or $-CH_2F$;

b) $R^1$ is alternatively hydrogen, Cl or F;

$R^3$, $R^4$, $R^5$, $R^6$ and $R^7$ are identical or different and are

a) hydrogen
b) a straight-chain or branched alkyl radical (with or without an asymnmetrical carbon atom) having 1 to 16 carbon atoms, where

b1) one or more nonadjacent and non-terminal $-CH_2-$ groups may be replaced by; -O-;
and/or
b2) one or two $-CH_2-$ groups may be replaced by -CH=CH-,

c) $R^4$ and $R^5$ together may alternatively be $-(CH_2)_4-$ or $-(CH_2)_5-$ if they are bonded to an oxirane, dioxolane, tetrahydrofuran, tetrahydropyran, butyrolactone or valerolactone system;

with the proviso that $R^3$ can only be hydrogen If $R^3$ is a substituents of one of the ring systems mentioned;

$M^1$ is a single bond;
$A^1$ is a single bond.
$X_1$, $Y_1$ fluorine

with the proviso that

i) $Y_2$, $X_2$, $X_3$, $X_4$ are hydrogen, or
ii) $Y_2$, $X_2$ are fluorine and $X_3$, $X_4$ are hydrogen
iii) $Y_2$, $R_1$ are fluorine and $X_2$, $X_3$, $X_4$ are hydrogen, or
iiii) $X_2$ is fluorine and $Y_2$, $X_3$, $X_4$ are hydrogen, $G_1$ is -CH=CH-, $R_1$ is H, Cl, F, $CF_3O$, $CH_2CHO$, $CF_3$, a linear alkyl radical having 1 to 5 carbon atoms and $R_2$ is a linear alkyl or alkoxy radical having 1 to 5 carbon atoms.

**3.** A liquid-crystal mixture comprising one or more compounds of the formula (I) as claimed in claim 1 or 2.

**4.** A liquid-crystal mixture as claimed in claim 3 which is chiral tilted smectic or nematic.

**5.** A liquid-crystal mixture as claimed in claim 4 which comprises from 0.01 to 80% by weight of one or more derivatives of the formula (I).

**6.** A switching or display device containing a liquid-crystal mixture as claimed in claim 5.

**7.** A switching and display device in the form of a ferroelectric switching and/or display device containing a chiral tilted smectic liquid-crystal mixture as claimed in claim 4, or in the form of an active-matrix display device containing a liquid-crystal mixture as claimed in claim 4, or in the form of an active-matrix display device containing a liquid-crystal mixture as claimed in claim 5, or in the form of a super-TFT (IPSI) display device containing a nematic liquid-crystal mixture as claimed in claim 4, or in the form of an ECB display device containing a nematic liquid-crystal mixture as claimed in claim 4.

**8.** A monostable ferroelectric active-matrix display containing a liquid-crystal mixture as claimed in claim 4.

**Revendications**

**1.** Dérivé fluoré du phénanthrène de formule (I),

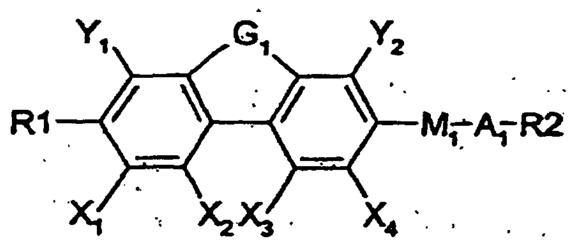

(I)

les symboles et les indices possèdent les significations suivantes :

G1 : représente un groupe -CH=CH- ou $-CH_2CH_2-$

$X_1$, $X_2$, $X_3$, $X_4$ : indépendamment les uns des autres représentent un atome de H ou de F, à condition que

a) $X_1$ et $X_2$ et $X_3$ et $X_4$ ne puissent pas représenter en même temps un atome de H
b) $X_1$ et $X_2$ et $X_3$ et $X_4$ ne puissent pas représenter en même temps un atome de F
c) au moins un X de ce groupe représente un atome de F ;

$Y_2$ : représente un atome de H ou de F ; $Y^1$ : représente un atome de F ;

$R^1$, $R^2$ identiques ou différents

a) représentent un reste alkyle linéaire ou ramifié (avec ou sans atome de carbone asymétrique) présentant 1 à 20 atomes de carbone, où aussi un ou plusieurs atomes de H peuvent être substitués par un atome de F et

a1) un ou plusieurs groupes $CH_2$ non adjacents et non terminaux peuvent être remplacés par -O-, -S-, -CO-O-, -O-CO-, -0-CO-O- ou -Si $(CH_3)_2$- et/ou
a2) un ou plusieurs groupes $CH_2$ peuvent être remplacés par -CH=CH-, -C≡C-, cyclopropane-1,2-diyle, 1,4-phénylène, 1,4-cyclohexylène ou 1,3-cyclopentylène et/ou

a3) le groupe terminal $CH_3$ peut être remplacé par un des groupes chiraux (optiquement actifs ou racémiques) suivants :

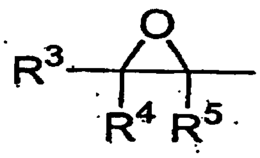

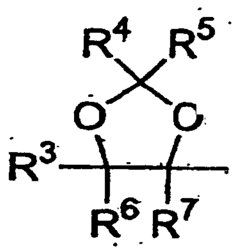

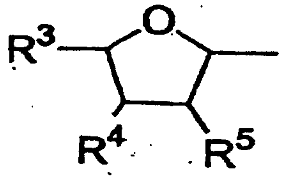

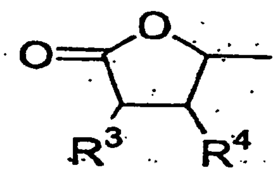

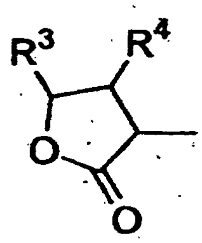

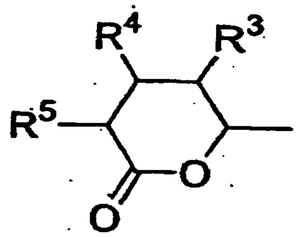

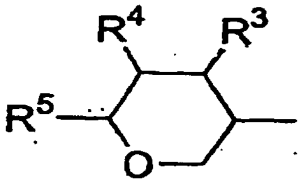

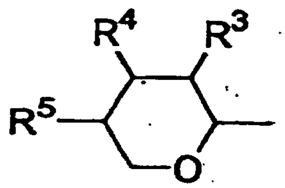

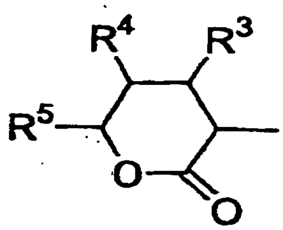

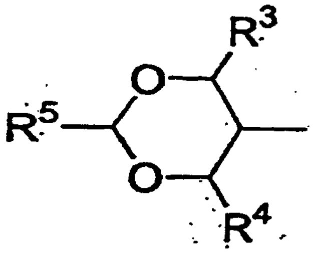

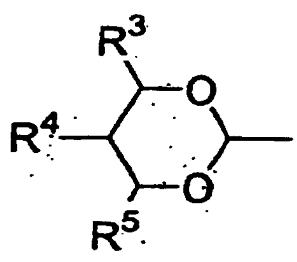

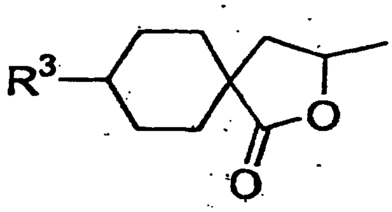

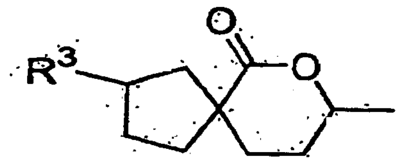
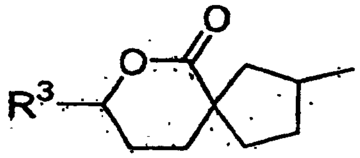
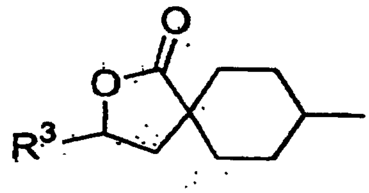

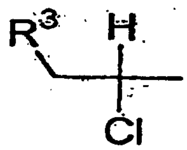
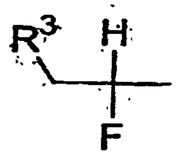
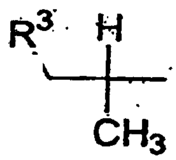
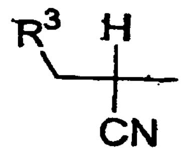

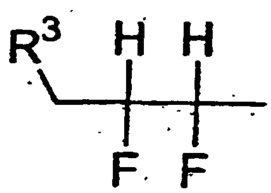

$R^1$ peut aussi représenter un atome d'hydrogène, un groupe $-OCF_3$, $-CF_3$, -CN, -F, -Cl, $-OCHF_2$, $-OCH_2F$, $-CHF_2$ ou $-CH_2F$ ;

b) $R^1$ représente aussi un atome d'hydrogène, de Cl ou de F,

$R^3$, $R^4$, $R^5$, $R^6$, $R^7$ identiques ou différents

a) représentent un atome d'hydrogène
b) représentent un reste alkyle linéaire ou ramifié (avec ou sans atome de carbone asymétrique) présentant 1 à 16 atomes de carbone,

b1) un ou plusieurs groupes $CH_2$ non adjacents et non terminaux peuvent être remplacés par -O- et/ou
b2) un ou deux groupes $CH_2$ peuvent être remplacés par un groupe -CH=CH-,

c) $R^4$ et $R^5$ conjointement représentent aussi $-(CH_2)_4-$ ou $-(CH_2)_5-$ lorsqu'ils sont liés à un système oxirane, dioxolane, tétrahydrofuranne, tétrahydropyrane, butyrolactone ou valérolactone ;

à condition que $R^3$ ne puisse représenter un atome d'hydrogène que si $R^3$ est un substituant d'un des systèmes cycliques indiqués ;

$M^1$ représente un groupe -CO-O-, $-CH_2-O-$, -CH=CH-, -C≡C-, $-CH_2CH_2-CO-O-$, $-CH_2CH_2CH_2CH_2-$, $CH_2CH_2CH_2O-$ et leurs configurations spéculaires ; ou une liaison simple ;

$A^1$ représente un groupe 1,4-phénylène, un ou plusieurs atomes d'hydrogène peuvent être remplacés par un groupe F, Cl et/ou CN, 1,4-cyclohexylène, un ou deux atomes d'hydrogène peuvent être remplacés par un groupe CN et/ou $CH_3$ et/ou F, naphtalène-2,6-diyle, un ou plusieurs atomes d'hydrogène pouvant être remplacés par un groupe F, Cl et/ou CN ; ou une

liaison simple.

2. Dérivé fluoré du phénanthrène selon la revendications 1 les symboles et les indices possèdent les significations suivantes :

| | |
|---|---|
| G1 : | représente un groupe $-CH=CH-$ ou $-CH_2CH_2-$, |
| $X_2$, $X_3$, $X_9$ : | indépendamment les uns des autres représentent un atome de H ou de F , |
| $Y_2$ : | à représente un atome de H ou de F, |
| $R^1$, $R^2$ | identiques ou différents |

a) représentent un reste alkyle linéaire ou ramifié (avec ou sans atome de carbone asymétrique) présentant 1 à 20 atomes de carbone, où aussi un ou plusieurs atomes de H peuvent être substitués par un atome de F et

a1) un ou plusieurs groupes $CH_2$ non adjacents et non terminaux peuvent être remplacés par -O-, -S-, -CO-O-, -O-CO-, -O-CO-O- ou $-Si(CH_3)_2-$ et/ou
a2) un ou plusieurs groupes $CH_2$ peuvent être remplacés par un groupe -CH=CH-, -C≡C-, cyclopropane-1,2-diyle, 1,4-phénylène, 1,4-cyclohexylène ou 1,3-cyclopentylène et/ou
a3) le groupe $CH_3$ terminal peut être remplacé par un des groupes chiraux (optiquement actifs ou racémiques) suivants :

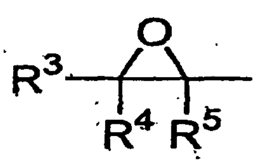
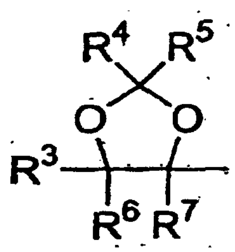
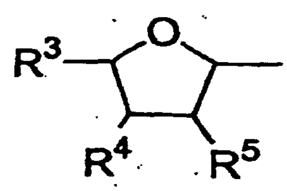

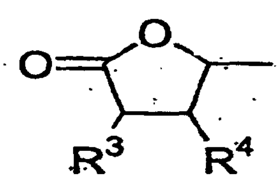
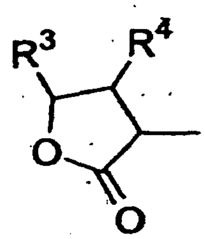
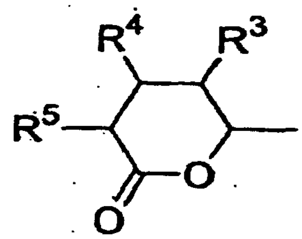

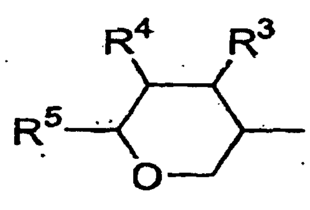
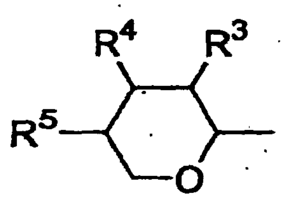
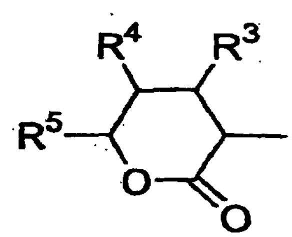

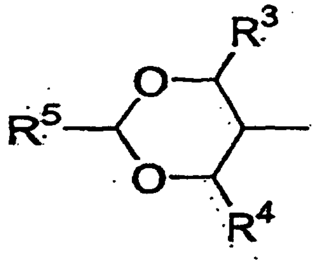
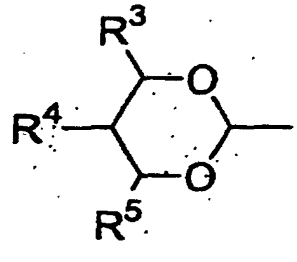
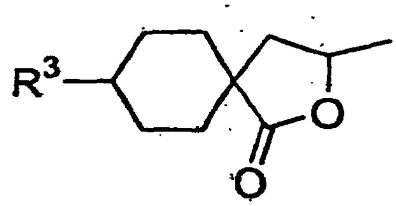

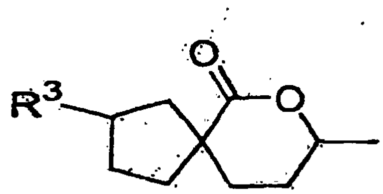
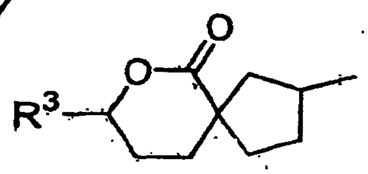
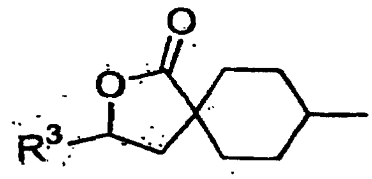

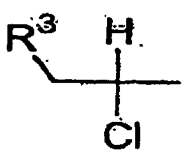
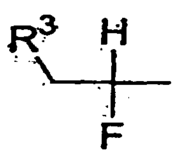
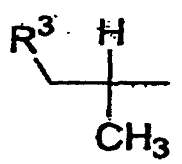
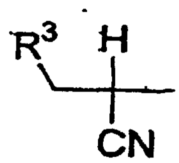

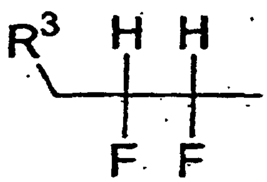

| | |
|---|---|
| $R^1$ | représente aussi un atome d'hydrogène, un groupe $-OCF_3$, $-CF_3$, -CN, -F, -Cl, $-OCHF_2$, $-OCH_2F$, $-CHF_2$ ou $-CH_2F$ ; |
| | b) $R^1$ représente aussi un atome d'hydrogène, de Cl ou de F, |
| $R^3$, $R^4$, $R^5$, $R^6$, $R^7$ | identiques ou différents |
| | a) représentent un atome d'hydrogène,<br>b) représentent un reste alkyle linéaire ou ramifié (avec ou sans atome de carbone asymétrique) présentant 1 à 16 atomes de carbone, |

b1) un ou plusieurs groupes $CH_2$ non adjacents et non terminaux peuvent être remplacés par -O- et/ou
b2) un ou deux groupes $CH_2$ peuvent être remplacés par un groupe -CH=CH-,

c) $R^4$ et $R^5$ conjointement représentent aussi - $(CH_2)_4$- ou -$(CH_2)_5$- lorsqu'ils sont liés à un système oxirane, dioxolane, tétrahydrofuranne, tétrahydropyrane, butyrolactone ou valérolactone ;

à condition que $R^3$ ne puisse représenter un atome d'hydrogène que si $R^3$ est un substituant d'un des systèmes cycliques indiqués ;

$M^1$ représente une liaison simple ;
$A^1$ représente une liaison simple ;
$X_1$, $Y_1$ représentent un atome de fluor

à condition que

i) $Y_2$, $X_2$, $X_3$, $X_4$ représentent un atome d'hydrogène, ou
ii) $Y_2$, $X_2$, représentent un atome de fluor et $X_3$, $X_4$ représentent un atome d'hydrogène, ou
iii) $Y_2$, $R_1$ représentent un atome de fluor et $X_2$, $X_3$, $X_4$ représentent un atome d'hydrogène, ou
iiii) $X_2$ représente un atome de fluor et $Y_2$, $X_3$, $X_4$ représentent un atome d'hydrogène, $G_1$ représente un groupe -CH=CH-, $R_1$ représente un atome d'hydrogène, de Cl, de F, un groupe $CF_3O$, $CH_2HO$, $CF_3$, un reste alkyle linéaire présentant 1 à 5 atomes de carbone et $R_2$ représente un reste alkyle ou alkoxy linéaire présentant 1 à 5 atomes de carbone.

**3.** Mélange de cristaux liquides, renfermant un ou plusieurs composés de formule (I) selon l'une des revendications 1 ou 2.

**4.** Mélange de cristaux liquides selon la revendication 3, **caractérisé en ce qu'**il est smectique incliné ou nématique.

**5.** Mélange de cristaux liquides selon la revendication 4, **caractérisé en ce qu'**il renferme de 0,01 à 80 % en masse d'un ou plusieurs dérivés de formule (I).

**6.** Dispositif de commutation ou d'affichage, renfermant un mélange de cristaux liquides selon la revendication 5.

**7.** Dispositif de commutation ou d'affichage sous forme

- d'un dispositif ferroélectrique de commutation et/ou d'affichage renfermant un mélange de cristaux liquides smectique incliné selon la revendication 4, ou
- d'un dispositif d'affichage à matrice active renfermant un mélange de cristaux liquides selon la revendication 4, ou
- d'un dispositif d'affichage super-TFT(IPSI) renfermant un mélange de cristaux liquides nématique selon la revendication 4, ou
- d'un dispositif d'affichage ECB renfermant un mélange de cristaux liquides nématique selon la revendication 4.

**8.** Dispositif d'affichage à matrice active ferroélectrique monostable, renfermant un mélange de cristaux liquides selon la revendication 4.